# EUROPEAN PATENT APPLICATION

(11) **EP 4 643 820 A1**
(43) Date of publication of application: **05.11.2025**
(21) Application number: 23910964.8
(22) Date of filing: 29.12.2023
(51) Int. Cl.: A61F 2/24

(54) **CONTROL SYSTEM AND DELIVERY SYSTEM FOR INTERVENTIONAL DEVICE**

(30) Priority: 30.12.2022 CN 202211739576; 30.12.2022 CN 202211738715; 30.12.2022 CN 202211735790
(71) Applicant: Peijia Medical Co., Ltd., Suzhou, Jiangsu 215000 (CN); Sierra Valve LLC, Wilmington, DE 19801 (US)
(72) Inventor: WANG, Kai, Suzhou, Jiangsu 215000 (CN); WANG, Dongyang, Suzhou, Jiangsu 215000 (CN); WANG, Jie, Suzhou, Jiangsu 215000 (CN); TAN, Jianfong, Suzhou, Jiangsu 215000 (CN); ZHANG, Yi, Suzhou, Jiangsu 215000 (CN)
(74) Representative: Isern Patentes y Marcas S.L.
(86) International application number: PCT/CN2023/143410
(87) International publication number: WO 2024/141051

(57) **Abstract**

The present disclosure provides a control system and a delivery system for an interventional device. The control system comprises a control handle that comprises a switching mechanism for toggling the transmission relationship between a knob and a drive shaft between relative rotation and concurrent rotation. This allows for easy switching between the clamping and separation processes through a simple switching action. The outer tube assembly, middle tube assembly, and inner tube assembly of the delivery system are designed as separate components that work in conjunction with each other, facilitating assembly, disassembly, cleaning, and replacement. The coordinated operation of the outer tube assembly and the middle tube assembly enables three-dimensional bending adjustment, facilitating path control within complex and curved cardiovascular passages. This design offers simple operation, high flexibility, and reduces the risk of trauma during surgical procedures.

## Description

### TECHNICAL FIELD

The present disclosure relates to the technical field of medical devices, and more particularly, to a control system and a delivery system for an interventional device.

### BACKGROUND

Common treatment approaches for mitral regurgitation and tricuspid regurgitation comprise artificial valve replacement surgery and valve repair and reconstruction. Artificial valve replacement surgery is typically an open-heart procedure during which the heart is stopped, and blood flow is managed by a cardiopulmonary bypass machine, exposing patients to many potential risks such as infection, stroke, and renal failure. Valve repair and reconstruction, on the other hand, is developed based on the principle of surgical edge-to-edge valve suture techniques. It involves delivering a valve clipping device to the heart in a minimally invasive, percutaneous, or transluminal manner, followed by using the device to repair the heart valve.

The valve clipping device used in cardiac interventional procedures needs to be guided to a specified location in the heart through a delivery assembly via a complex and curved cardiovascular pathway. This requires precise path control during delivery and manipulation of the valve clipping action by the device. Once the valve is securely clipped and fixed, the device remains in the heart as an implant, while the delivery system needs to be relatively detached from the valve clipping device via a clutch mechanism.

The drawbacks of the control systems and delivery systems for interventional devices in the existing technology are as follows: (1) The control system requires different operational methods to achieve the clipping and separation processes, and switching between these two processes is complex. Therefore, there is a need for a mechanism that facilitates easy switching between the clipping and separation processes, allowing the same operational technique to be used for different control processes after switching; (2) Operators cannot visually ascertain the degree of opening and closing of the closure components of the valve clipping device from a remote location, necessitating the use of additional in-vivo imaging equipment for observation. In-vivo imaging equipment can be harmful to the human body, and improper operation may lead to excessive rotation angles, causing the closure members to exceed their deployed limits. Hence, there is a need for a control system that provides clear indications of the clipping degree to address these technical issues; (3) The layout of the tubing, manipulation shafts, and manipulation wires in the control system is chaotic, resulting in poor structural stability; (4) The delivery system lacks flexibility and has difficulty navigating through curved pathways, potentially causing vascular injury.

### SUMMARY

In order to address the aforementioned technical problems, this application provides a control system and a delivery system for an interventional device.

Specifically, the following solutions are shown as below.

In a first aspect, this application provides a control system for an interventional device, comprising a control handle. The control handle comprises:
a control assembly, which comprises a manipulation shaft for controlling a distal instrument and a driving component for driving the manipulation shaft; the driving component comprises a drive shaft, which comprises a control shaft connection end at its distal end; the control shaft connection end is capable of transmitting torque and axial force to the manipulation shaft;
a housing assembly, which comprises an outer housing. The outer housing comprises a through-hole at its distal end for allowing an inner tube assembly to extend out of the outer housing, as well as a knob annular groove at its proximal end;
the driving component further comprises a knob and a switching mechanism; the knob is rotationally connected relative to the knob annular groove, the drive shaft comprises a threaded connection portion at its proximal end, and the knob is provided with an internal thread that matches the threaded connection portion, the switching mechanism is configured to toggle the transmission relationship between the knob and the drive shaft between relative rotation and concurrent rotation.

Further preferably, the switching mechanism comprises a detent block. The detent block comprises a first engagement structure, and the outer surface of the knob is provided with a second engagement structure. The detent block is sleeved over the drive shaft and is capable of sliding relative to the drive shaft between a first position and a second position.

When the detent block is located at the first position, the first engagement structure engages with the second engagement structure to restrict relative rotation between the knob and the detent block.

When the detent block is located at the second position, the first engagement structure disengages from the second engagement structure, allowing the knob to rotate relative to the detent block.

Further preferably, one of the first engagement structure and the second engagement structure is a key structure, while the other is a keyway structure. The key structure and the keyway structure are coupled at the first position to restrict relative rotation between the knob and the detent block_{∘}

Further preferably, the switching mechanism also comprises a clutch block and a spring. The outer housing is provided with a gear block annular groove that restricts the rotation of the detent block and guides the axial sliding of the detent block relative to the outer housing.

The proximal end of the clutch block is provided with a first compression structure, and the distal end of the detent block is provided with a second compression structure. The spring applies a biasing force to the clutch block, causing the first compression structure to press against the second compression structure.

Further preferably, the switching mechanism also comprises a toggle plate that is axially fixed to the detent block. The toggle plate comprises a first snap, and the outer housing is provided with a second snap that matches the first snap. When the toggle plate moves the detent block to the second position, the first snap engages with the second snap to restrict the detent block from moving back to the first position.

Further preferably, the first compression structure and the second compression structure are configured such that when the detent block rotates, the second compression structure transmits a biasing force to the first compression structure, driving the clutch block to move distally.

Further preferably, the second compression structure is a convex tooth, and the first compression structure is a latching tooth that matches the convex tooth.

Further preferably, the drive shaft comprises a transmission cavity section with a transmission guide face. The detent block is provided with a gear guide face that matches the transmission guide face. Whether the detent block is in the first position or the second position, the gear guide face is in contact with the transmission guide face to restrict the drive shaft from moving relative to the detent block.

Further preferably, a manipulation shaft positioning sleeve is fixedly sleeved on the proximal end of the manipulation shaft. The control shaft connection end comprises a shaped elongated hole, and the outer surface of the manipulation shaft positioning sleeve matches the inner surface of the shaped elongated hole to restrict relative rotation between the manipulation shaft and the drive shaft.

The manipulation shaft positioning sleeve and the shaped elongated hole are arranged to slide axially relative to each other. The length of the shaped elongated hole is greater than that of the manipulation shaft positioning sleeve. The inner diameter of the manipulation shaft annular groove is smaller than the maximum outer diameter of the manipulation shaft positioning sleeve.

Further preferably, a floating elastic member is disposed between the proximal end of the manipulation shaft positioning sleeve and the bottom surface of the shaped elongated hole.

Further preferably, the floating elastic member is a spring. When the floating elastic member is in its natural state, the distal end of the manipulation shaft positioning sleeve is located within the shaped elongated hole.

Further preferably, the housing assembly comprises an outer housing and a moving seat positioned within the outer housing. The moving seat comprises an inner cavity, and the control shaft connection end is located within the inner cavity of the moving base, with the ability to rotate relative to the moving seat.

Further preferably, the moving seat also comprises an inner guide face within its inner cavity. The moving seat is provided with a first observation window, and the outer housing is provided with a second observation window corresponding to the first observation window.

An indicating part is provided, which features a threaded characteristic. The indicating part is threadedly engaged with the control shaft connection end, and its outer surface is slidably connected to the inner guide face while being relatively fixed in the rotational direction. The indicating part serves an indicative function in conjunction with the first and second observation windows.

Further preferably, the inner cavity of the moving base comprises an inner end surface that cooperates with both ends of the control shaft connection end to confine it within the inner cavity of the moving base.

Further preferably, the moving seat is a split-type, detachable housing.

Further preferably, the moving seat comprises a manipulation shaft annular groove and a drive shaft annular groove, which are coaxially arranged. The inner diameter of the manipulation shaft annular groove is greater than or equal to the outer diameter of the manipulation shaft, while the inner diameter of the drive shaft annular groove is larger than the outer diameter of the drive connection section of the drive shaft but smaller than the outer diameter of the control shaft connection end.

Further preferably, the moving seat also comprises an outer guide face. The outer housing comprises a moving seat guide rail, with which the outer guide face slidably cooperates.

Further preferably, a positioning and guiding mechanism is provided. The positioning and guiding mechanism comprises an inner tube assembly, which comprises an inner tube and a sleeve for guiding the manipulation wire. The inner tube consists of an inner layer and an outer layer. The inner layer is provided with a cavity for the manipulation shaft to pass through, while the outer layer is provided with a channel for the manipulation wire to pass through. The proximal end of the inner layer extends beyond the outer layer, and an inner tube positioning sleeve is fixedly sleeved over at least part of the extending section.

The housing assembly comprises an outer housing and a 8fixed seat positioned within the outer housing. The fixed seat comprises a positioning sleeve slot and sleeve guide slots on both sides of the positioning sleeve slot. The two sleeve guide slots taper from the proximal end to the distal end. The positioning sleeve slot matches the inner tube positioning sleeve, and the sleeve guide slots match the sleeve.

Further preferably, the distal end of the fixed seat is also provided with an annular groove for the inner layer to pass through. The annular groove is in communication with the positioning sleeve slot, and the inner diameter of the annular groove is larger than that of the inner layer but smaller than that of the inner tube positioning sleeve.

Further preferably, the fixed seat adopts a split-type, detachable structure.

Further preferably, the outer housing is also equipped with a guide rail. The inner tube assembly further comprises a push-pull rod slidably disposed on the guide rail, with the push-pull rod being fixedly connected to the distal end of the manipulation wire.

Further preferably, the inner tube assembly also comprises a support tube disposed within the inner cavity of the push-pull rod, with the support tube being fixedly connected to the sleeve.

In a second aspect, this application provides a delivery system for an interventional device, comprising the aforementioned control system, a guiding catheter assembly, a middle tube assembly, an inner tube assembly, and a manipulation shaft.

The guiding catheter assembly comprises an outer tube assembly, which comprises an outer tube body. An outer tube deflection member is disposed within the outer tube body and is configured to control the deflection of at least part of the outer tube body;

The middle tube assembly comprises a middle tube body, within which a middle tube deflection member is disposed. The middle tube deflection member is configured to control the deflection of at least part of the middle tube body. The middle tube body is capable of accommodating the inner tube, and the outer tube body is capable of accommodating the middle tube body.

The inner tube assembly comprises an inner tube, within which a manipulation wire is disposed. The manipulation wire is configured to control the capturing action of the capturing members of a valve clamping device.

The manipulation shaft is inserted through the cavity of the inner tube. The distal end of the manipulation shaft is provided with a connecting portion that is connected to the valve clamping device. The manipulation shaft is capable of rotating relative to the inner tube to control the opening and closing of the closure member of the valve clamping device.

Further preferably, the outer tube body is provided with an inner liner layer and an outer tube body coating layer from the inside out along its radial direction. At least one outer tube support layer is embedded within the outer tube body coating layer. At least one outer tube deflection member liner tube is disposed within the outer tube body coating layer, and the outer tube deflection member is housed within the outer tube deflection member liner tube.

Further preferably, the outer tube body comprises, from the distal end to the proximal end, a deflection tip, a pull-ring section, a deflection section, and a main body section. The pull-ring section is connected to the distal end of the outer tube deflection member, while the proximal end of the outer tube deflection member is connected to an outer tube handle. The hardness of the deflection section is less than that of the main body section;

Preferably, at least one transition section is provided between the deflection section and the main body section. The hardness of the transition section is greater than that of the deflection section but less than that of the main body section.

Further preferably, the outer tube handle is equipped with an outer tube deflection assembly, which comprises an outer tube knob and an outer tube threaded member. The outer tube threaded member is sleeved over the outer tube body and connected to the outer tube deflection member. The outer tube knob is configured to rotate and drive the outer tube threaded member to move axially, thereby pulling the outer tube deflection member.

Further preferably, the middle tube body is provided with a middle tube inner liner layer and a middle tube body coating layer along its radial direction. At least one middle tube support layer is embedded within the middle tube body coating layer. At least one middle tube deflection member liner is disposed within the middle tube body coating layer, and the middle tube deflection member is housed within the middle tube deflection member liner.

Further preferably, the middle tube body comprises, from the distal end to the proximal end, a middle tube pull-ring section, a middle tube deflection section, a middle tube compliant section, and a middle tube main body section. The middle tube pull-ring section is connected to the distal end of the middle tube deflection member, while the proximal end of the middle tube deflection member is connected to the middle tube handle. The hardness of the middle tube deflection section is less than that of the middle tube compliant section, and the hardness of the middle tube compliant section is less than that of the middle tube main body section.

Preferably, at least one middle tube transition section is provided between the middle tube main body section and the middle tube compliant section, as well as between the middle tube compliant section and the middle tube deflection section. The hardness of the middle tube transition section is less than that of the middle tube main body section.

Further preferably, the middle tube handle is equipped with a middle tube deflection assembly, which comprises a middle tube knob and a middle tube threaded member. The middle tube threaded member is sleeved over the middle tube body and connected to the middle tube deflection member. The middle tube knob is configured to rotate and drive the middle tube threaded member to move axially, thereby pulling the middle tube deflection member.

Further preferably, the inner tube is connected to a control handle within the control system. The control handle is provided with a joystick that is connected to the manipulation wire.

Further preferably, the inner tube is composed of an inner layer and an outer layer from the inside out along its radial direction. At least one channel is disposed within the outer layer, and the manipulation wire is housed within the at least one channel.

Further preferably, the guiding catheter assembly also comprises a guide catheter that is detachably positioned within the outer tube body. The guide catheter comprises a main body section and a guiding head that are connected to each other. The outer diameter of the guiding head gradually decreases from the end connected to the main body section to the end away from the main body section.

As mentioned above, this application offers the following beneficial effects.

The control system of this application enables seamless switching between the gripping and separation processes through simple switching actions, making it more convenient and reliable compared to the existing driving methods of valve gripping mechanisms.

This application leverages the rotational characteristics of the drive shaft, in conjunction with the special transmission relationship between the indicating part and the moving seat, allowing operators to visually assess, through an observation window on the housing assembly, the opening and closing degrees of the closure members of the valve clamping device at the distal end, as well as determine the engagement and disengagement status between the valve clamping device and the control system. This eliminates the need for additional in-vivo imaging equipment and prevents technical issues such as excessive rotational angles that might cause the closure members to exceed their deployment limits.

The housing assembly of this application adopts a guided and fixed approach, converging the manipulation shaft and manipulation wire into the inner tube before entering the human body, resulting in a compact and reasonable layout with stable and reliable structure.

The manipulation shaft of this application can float axially relative to the drive shaft when subjected to compression, thereby resolving quality issues caused by excessive compression of the manipulation shaft. The floating connection method of this application ensures that the axial displacement of the manipulation shaft is not affected by the drive shaft during the opening and closing of the closure members. Additionally, a floating elastic member, such as a spring, can be placed between the proximal end of the manipulation shaft positioning sleeve and the bottom surface of an irregularly shaped elongated hole. When the floating elastic member is in its natural state, the distal end of the manipulation shaft positioning sleeve is located within the irregularly shaped elongated hole. After incorporating the floating elastic member, a pre-compression force is established between the joystick and the drive rod through the transmission of the manipulation shaft, preventing accidental disengagement caused by unexpected impacts.

The outer tube assembly, middle tube assembly, and inner tube assembly of the delivery system in this application are designed as separate components that work in conjunction with each other, facilitating easy assembly, disassembly, cleaning, replacement, and reassembly. The cooperation between the outer tube assembly and the middle tube assembly enables three-dimensional deflection, facilitating path control within complex and curved cardiovascular channels. This design offers simplicity in operation, high flexibility, and reduces damage during surgical procedures.

### BRIEF DESCRIPTION OF THE DRAWINGS

To more clearly illustrate the technical solutions of the present disclosure, a brief introduction to the accompanying drawings required for the description of the embodiments or the prior art will be provided below. It is evident that the drawings described below are merely some embodiments of the present disclosure. For those skilled in the art, other drawings can also be obtained based on these drawings without the need for inventive effort.
FIG. 1 is a schematic perspective view of the present application;
FIG. 2 is a schematic perspective view of the present application from another angle;
FIG. 3 is a schematic structural view of FIG. 1 with half of the outer housing removed;
FIG. 4 is a schematic structural view of FIG. 3 with some components removed;
FIG. 5 is a front cross-sectional view of the present application;
FIG. 6 is a partially enlarged view of FIG. 5;
FIG. 7 is a partially enlarged view of FIG. 6 at location A;
FIG. 8 is a partially enlarged view of FIG. 5 at another location;
FIG. 9 is a cross-sectional view of the present application in another direction;
FIG. 10 is a schematic structural view showing the position of the indicating part when the closure members of the valve clamping device are in a closed state;
FIG. 11 is a schematic structural view showing the position of the indicating part when the closure members of the valve clamping device are in an inverted state;
FIG. 12 is a schematic structural view showing the position of the indicating part when the closure members of the valve clamping device are in a 108° deployed state;
FIG. 13 is a schematic structural view of the moving seat when the capturing members of the valve clamping device are retracted and the closure members are in a 120° deployed state;
FIG. 14 is a schematic structural view showing the position of the indicating part when the capturing members of the valve clamping device are retracted and the closure members are in a 120° deployed state;
FIG. 15 is a schematic structural view showing the position of the indicating part when the capturing members of the valve clamping device are released and the closure members are in a 120° deployed state;
FIG. 16 is a schematic structural view showing the position of the indicating part when the capturing members of the valve clamping device are released and the closure members are in a 60° deployed state;
FIG. 17 is a cross-sectional view of the present application when the gear block is in a second position;
FIG. 18 is a schematic perspective view of Figure 17;
FIG. 19 is a schematic structural view showing the release state of the valve clamping device corresponding to Figure 18;
FIG. 20 is a schematic structural view of the outer housing of the present application;
FIG. 21 is a schematic structural view of the fixed seat of the present application from two perspectives;
FIG. 22 is a schematic structural view of the moving seat of the present application from two perspectives;
FIG. 23 is a partial schematic structural view of the inner tube assembly of the present application;
FIG. 24 is a schematic view of the drive shaft of the present application from different perspectives;
FIG. 25 is a schematic structural view of the indicating part of the present application;
Figure 26 is a partially enlarged view of the floating mechanism of the present application;
FIG. 27 is a schematic view of the detent block of the present application from four different perspectives;
FIG. 28 is a schematic view of the toggle plate of the present application from two perspectives;
FIG. 29 is a schematic view of the knob of the present application from two perspectives;
FIG. 30 is a schematic structural view of the threaded block of the present application;
FIG. 31 is a schematic structural view of the clutch block of the present application from two perspectives;
FIG. 32 is a schematic structural view of the delivery system of the present application;
FIG. 33 is a schematic structural view of the outer tube assembly of the present application;
FIG. 34 is a cross-sectional view of the outer tube body in one embodiment of the present application;
FIG. 35 is a cross-sectional view of the outer tube body in an embodiment of the present application;
FIG. 36 is a schematic structural view of the outer tube body of the present application;
FIG. 37 is an axial cross-sectional view of the outer tube body of the present application;
FIG. 38 is a cross-sectional view of the outer tube handle of the present application;
FIG. 39 is a schematic structural view of the guide catheter of the present application;
FIG. 40 is a schematic structural view of the inner tube assembly of the present application;
FIG. 41 is a schematic structural view showing the connection between the middle tube assembly, inner tube assembly, and valve clamping device of the present application;
FIG. 42 is a cross-sectional view of the inner tube in one embodiment of the present application;
FIG. 43 is a cross-sectional view of the inner tube in another embodiment of the present application;
FIG. 44 is a cross-sectional view of the inner tube in an embodiment of the present application;
FIG. 45 is a schematic structural view of the middle tube assembly of the present application;
FIG. 46 is a schematic structural view showing the cooperation between the middle tube assembly and the inner tube assembly of the present application;
FIG. 47 is a schematic structural view of the middle tube body of the present application;
FIG. 48 is an axial cross-sectional view of the middle tube body of the present application;
FIG. 49 is a schematic three-dimensional deflection view showing the cooperation between the outer tube assembly, middle tube assembly, and inner tube assembly of the present application.
reference numerals:
housing assembly 1, outer housing 11, fluid injection cavity 101, drive cavity 102, moving seat guide rail 111, guide rail 112, clutch block annular groove 113, gear block annular groove 114, knob annular groove 115, second snap 116, second observation window 117, fixed seat 12, positioning sleeve slot 121, sleeve guide slot 122, annular groove 123, moving seat 13, inner cavity 131, inner end face 1311, inner guide face 132, outer guide face 133, manipulation shaft annular groove 134, drive shaft annular groove 135, first observation window 136, inner tube assembly 2, control handle 20, inner tube 21, inner layer 211, cavity 2111, outer layer 212, inner tube positioning sleeve 22, push-pull rod 23, sleeve 24, support tube 25, main tube body layer 213, tube body coating layer 214, channel 215, reinforcing tube layer 218, elastic tube layer 219, joystick 221, control assembly 3, manipulation shaft 31, connection portion 311, spring tube layer 312, manipulation main body tube 313, heat-shrink tube layer 314, manipulation shaft positioning sleeve 32, floating elastic member 320, manipulation wire 33, drive shaft 34, control shaft connection end 341, fluid injection cavity section 342, transmission cavity section 343, transmission guide face 344, threaded block 345, locknut 346, irregular shaped hole of threaded block 3451, conduction face 3431, knob 35, second snap-fit structure 351, handwheel 352, internal thread of knob 353, recessed annular groove feature 354, clutch block 36, first compression structure 361, spring 362, detent block 37, first snap-fit structure 371, second compression structure 372, gear guide face 374, toggle plate annular groove 375, toggle plate 38, first snap 381, inner ring of toggle plate 382, lever 383, sliding button 39, indicating part 4, valve clamping device 5, closure member 52, capturing member 51, outer tube assembly 6, outer tube body 61, outer tube deflection member 611, inner lining layer 612, outer tube body coating layer 613, outer tube support layer 614, outer tube deflection member liner tube 615, outer tube handle 62, outer tube deflection assembly 621, outer tube Luer three-way valve 622, outer tube hemostasis valve 623, outer tube knob 6211, outer tube threaded member 6212, knob fixing sleeve 6213, internally threaded member 6214, deflection tip 63, pull-ring section 64, deflection section 65, transition section 66, first transition section 661, second transition section 662, main body section 67, outer tube stress diffusion tube 68, outer tube radiopaque marker 69, middle tube assembly 7, middle tube body 71, middle tube deflection member 711, middle tube handle 72, middle tube deflection assembly 721, first middle tube Luer three-way valve 722, second middle tube Luer three-way valve 723, middle tube pull-ring section 73, middle tube deflection section 74, middle tube compliant section 75, middle tube transition section 750, first middle tube transition section 751, second middle tube transition section 752, third middle tube transition section 753, middle tube main body section 76, middle tube stress diffusion tube 77, protective sheath 78, middle tube radiopaque marker 79, guide catheter 8, main body portion 81, guide head 82, Luer head 83.

### DETAILED DESCRIPTION

The technical solutions in the embodiments of the present disclosure will be described clearly and completely below with reference to the accompanying drawings in the embodiments of the present disclosure. It is evident that the described embodiments are merely a part of the embodiments of the present disclosure, rather than all of them. All other embodiments obtained by those of ordinary skill in the art based on the embodiments of the present disclosure without any inventive effort shall fall within the protection scope of the present disclosure.

It should be noted that in this embodiment, the term "proximal end" refers to the direction closer to the operator, while the term "distal end" refers to the direction away from the operator. The central axis of the control handle is used as the reference axis.

### Examples

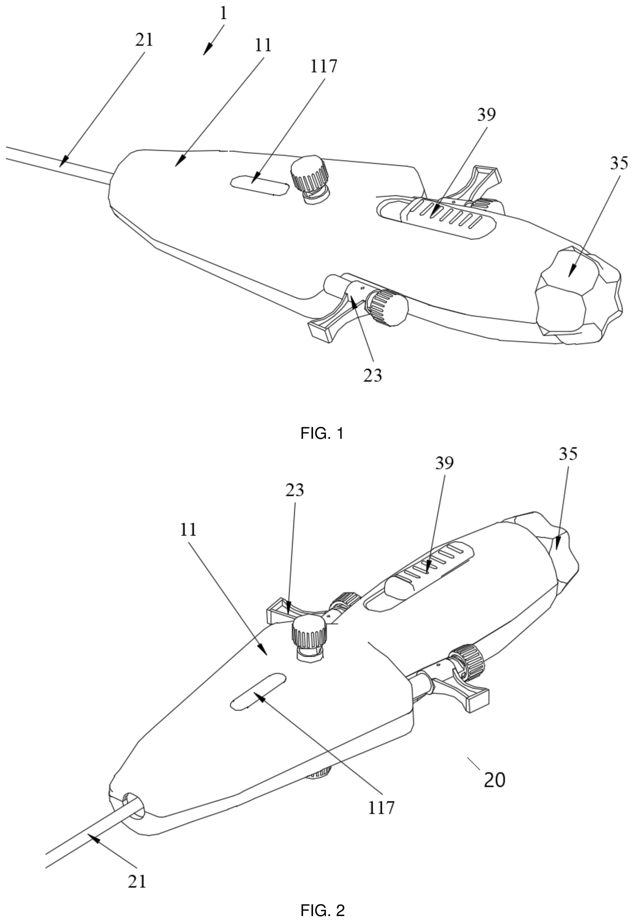

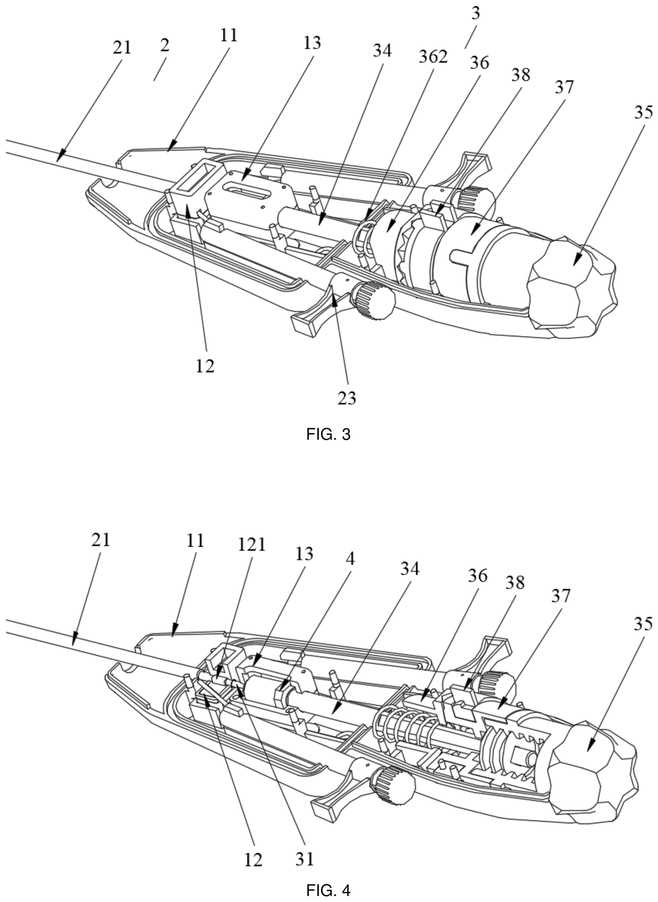

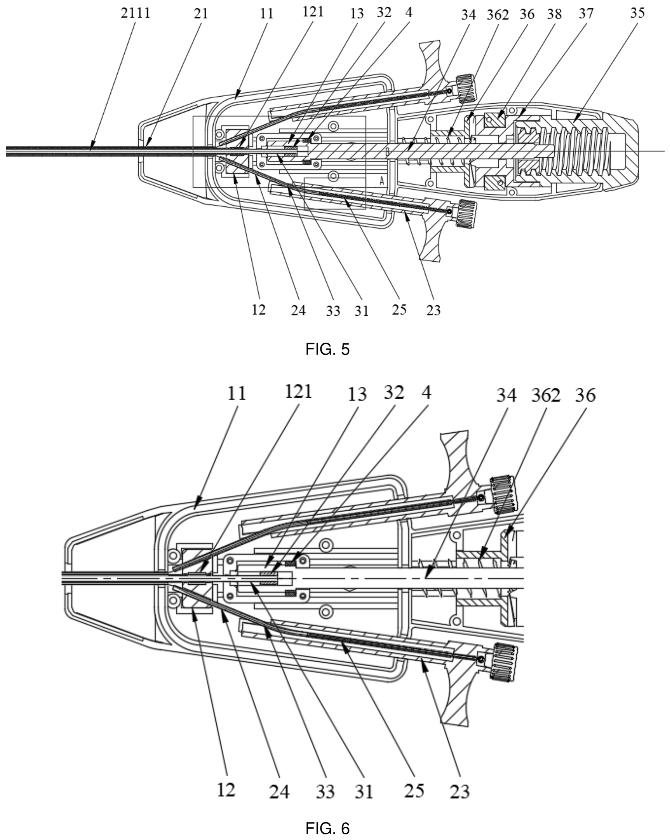

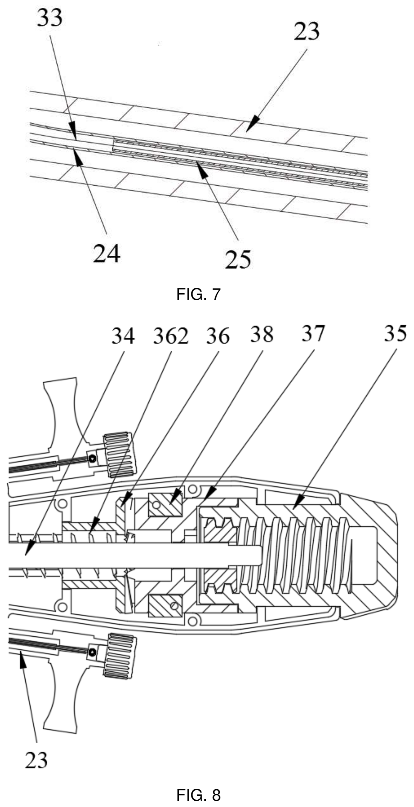

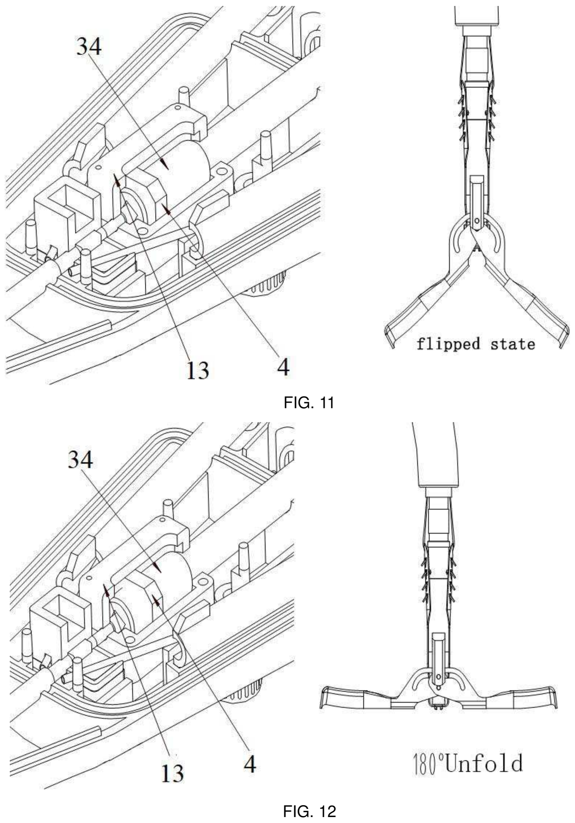

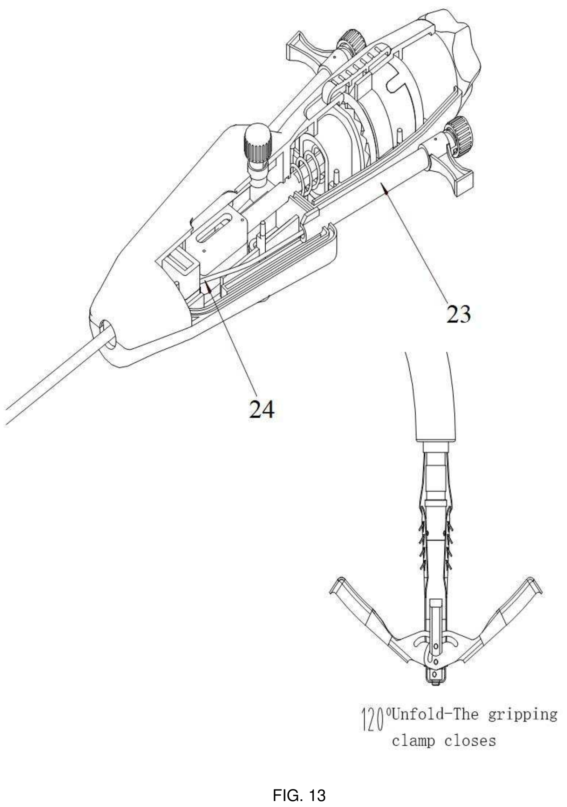

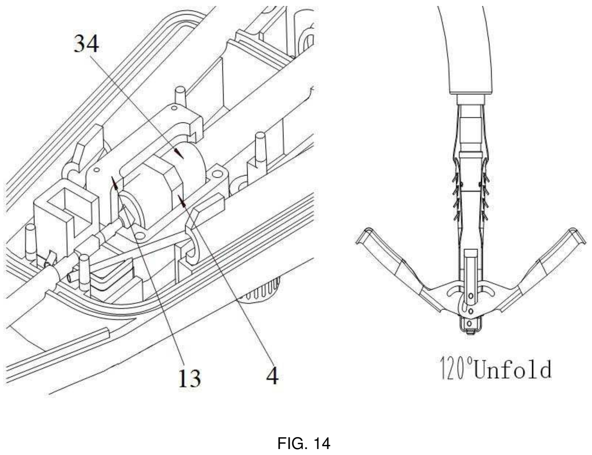

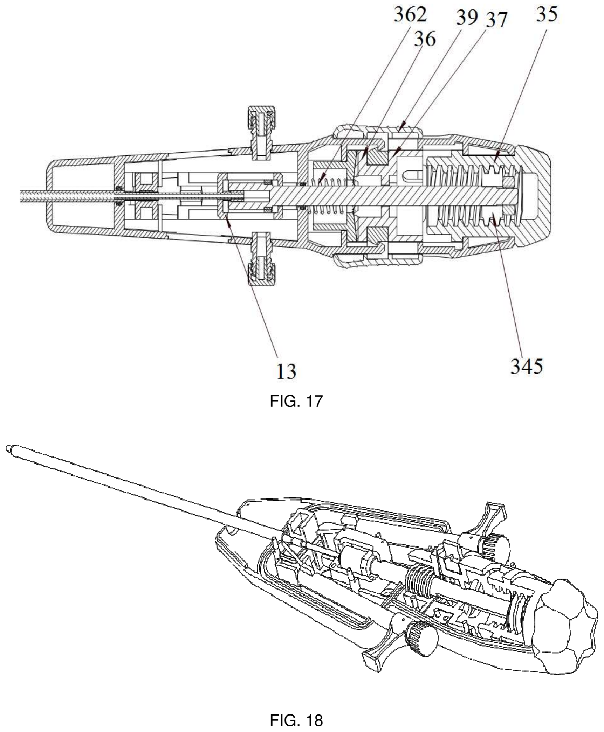

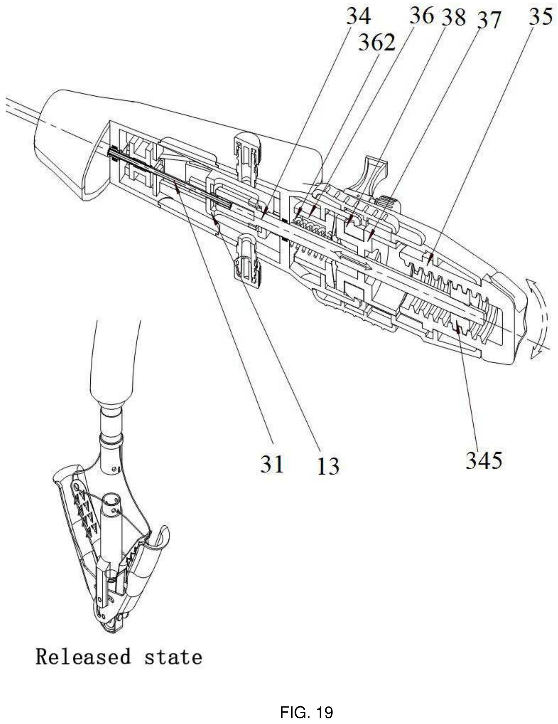

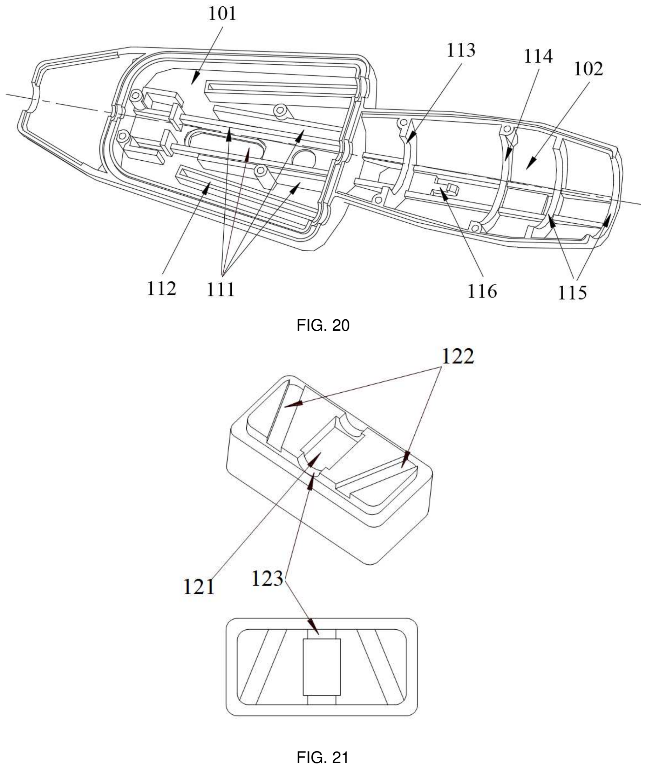

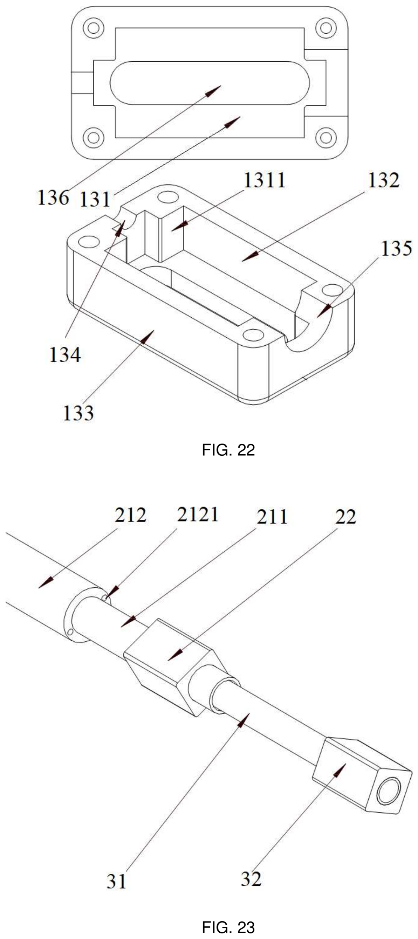

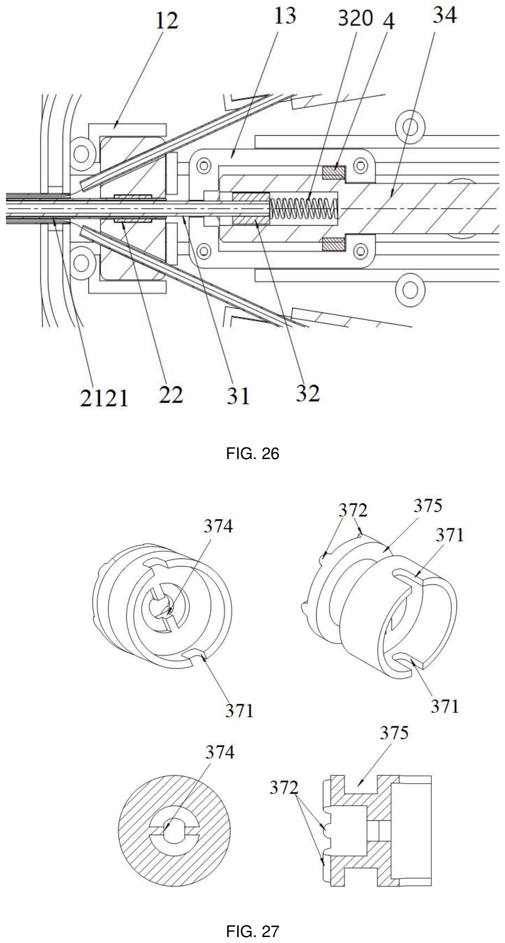

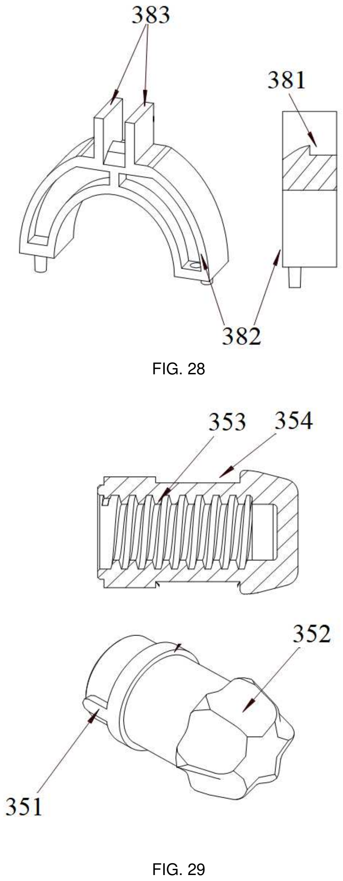

Referring to Figures 1-31, an embodiment of the present application provides a control system for an interventional device, specifically for the operational control of a valve clamping device. The valve clamping device is delivered to a designated position in the heart via a delivery system and remains in the heart as an implant after clamping and securing the damaged valve tissue. The control system, however, needs to be relatively separated from the valve clamping device through a clutch mechanism. The objective of the control system in this application is to provide the driving force for controlling the opening and clamping actions of the closure members of the valve clamping device, as well as the driving force required to fully separate all connecting structures that transmit relevant driving forces when the valve clamping device is separated from the control system. Specifically, the valve clamping device 5 comprises a pair of closure members 52 and a pair of capturing members 51 corresponding to each closure member 52. The closure members 52 achieve opening and closing through a drive assembly, while the capturing members 51 achieve opening and closing through manipulation wires 33. When clamping tissue, the cooperation between the inner sides of the closure members 52 and the outer sides of the capturing members 51 facilitates the clamping action. After completing the clamping, the control system needs to detach from the valve clamping device 5, leaving it inside the human body.

To achieve the aforementioned two processes, the control systems in the prior art require different operational means for the clamping and separation processes, and the switching between these two processes is relatively complex. Therefore, it is necessary to provide a mechanism that facilitates the switching between the clamping and separation processes, enabling different control processes to be achieved using the same operational method after switching. The present application can accomplish the switching between the clamping and separation processes through a simple switching action, offering a more convenient and reliable operation compared to the existing driving methods for valve clamping mechanisms. The specific structure for achieving this is as follows:
In this embodiment, the control system comprises a control handle 20 and an inner tube assembly 2. The control handle 20 comprises a housing assembly 1, a control assembly 3, and an indicating part 4. Among them, the control assembly comprises a manipulation shaft 31 for controlling the distal instrument, manipulation wires 33, and a drive assembly for driving the manipulation shaft 31. The manipulation shaft 31 is configured to connect to a threaded mechanism at the distal end, and the opening and closing of the closure members 52 are achieved through the rotation of the manipulation shaft 31. The specific driving process is prior art and will not be elaborated on here. The drive assembly comprises a drive shaft 34, which comprises a control shaft connection end 341 at the distal end. The control shaft connection end 341 is capable of transmitting torque and axial force to the manipulation shaft 31. When the drive shaft 34 rotates, it synchronously drives the manipulation shaft 31 to rotate, thereby controlling the opening and closing of the closure members 52. In some implementations, the control shaft connection end 341 can transmit axial tension or compression to the manipulation shaft 31. For example, a fixed stopper can be set inside the control shaft connection end 341 of the drive shaft 34 to block the movement of the manipulation shaft positioning sleeve 32, thus transmitting axial tension or compression to the manipulation shaft 31.

The housing assembly 1 comprises an outer housing 11 and a moving seat 13 located within the outer housing 11. The moving seat 13 is a split-type detachable shell. During use, the slots of the two moving seats 13 are aligned to form a closed box. The split-type design of the moving seat 13 facilitates assembly and disassembly. The moving seat 13 comprises a manipulation shaft annular groove 134 and a drive shaft annular groove 135. These two annular grooves are respectively set on the thin walls of the box at the inner and outer end faces on both sides, and they are coaxial with each other. After being assembled with the outer housing 11, they are also coaxial with the reference axis.

Specifically, the moving seat 13 comprises an outer guide face 133, and the outer housing 11 comprises a moving seat guide rail 111. The outer guide face 133 slidably engages with the moving seat guide rail 111. Specifically, the main body of the outer housing 11 is a shell that contains at least a fluid injection cavity 101 and a drive cavity 102. Inside the fluid injection cavity 101, several ribs parallel to the reference axis are arranged to serve as tracks for the moving seat 13, limiting the movement direction of the moving seat 13 to be parallel to the reference axis. Preferably, the side faces of the two farthest parallel ribs engage with the outer guide faces 133 of the moving seat 13, and the distance between the two parallel ribs is equal to the distance between the two outer guide faces 133 of the moving seat 13, with a clearance fit between them. The top faces of the two nearest parallel ribs engage with the outer guide faces 133 of the moving seat 13, and the distance between the two parallel ribs is less than the distance between the two outer guide faces 133 of the moving seat 13. When the moving seat 13 slides towards the proximal end relative to the outer housing 11, it can drive the manipulation shaft 31 to slide together, thereby achieving the separation of the transmission mechanism of the distal instrument. To realize the switching between the opening/closing control and the separation control of the closure members 52, in this embodiment, the drive assembly further comprises a knob 35 and a switching mechanism. The knob 35 is rotatably connected to the outer housing 11. The drive shaft 34 comprises a threaded connection portion at the proximal end, and the knob 35 is equipped with an internal thread that matches the threaded connection portion. The switching mechanism is configured to switch the transmission relationship between the knob 35 and the drive shaft 34 between relative rotation and co-rotation.

To achieve the switching between the opening/closing control and the separation control processes of the closure members 52, the drive assembly 3 comprises a knob 35 and a switching mechanism. The switching mechanism comprises a detent block 37, which comprises a first snap-fit structure 371. The outer surface of the knob 35 is provided with a second snap-fit structure 351. The detent block 37 is sleeved over the drive shaft 34 and is capable of sliding relative to the drive shaft 34 between a first position and a second position. When the detent block 37 is in the first position, the first snap-fit structure 371 engages with the second snap-fit structure 351 to restrict relative rotation between the knob 35 and the detent block 37. When the detent block 37 is in the second position, the first snap-fit structure 371 disengages from the second snap-fit structure 351, allowing the knob 35 to rotate relative to the detent block 37.

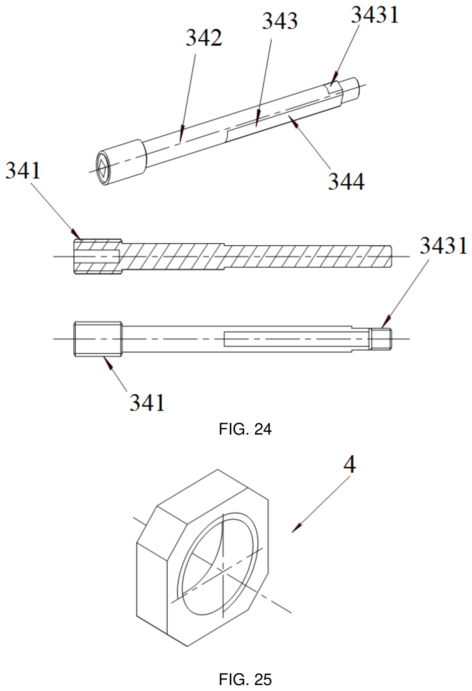

In order to specifically illustrate the implementation process of the switching mechanism, referring to Figure 24 of the accompanying drawings, the drive shaft 34 can be divided into a fluid injection cavity section 342 and a transmission cavity section 343 based on different cross-sections. The cross-section of the fluid injection cavity section 342 is circular, while the cross-section of the transmission cavity section 343 is non-circular and irregular. The end of the fluid injection cavity section 342 is provided with a control shaft connection end 341, whose cross-sectional diameter is larger than that of the fluid injection cavity section 342. The outer side of the control shaft connection end 341 is provided with threaded features, and its end face is equipped with a non-circular, irregularly shaped elongated hole. The cross-section of the irregularly shaped elongated hole of the control shaft connection end 341 may or may not be the same as the outer axial cross-sectional shape of the positioning sleeve, as long as it ensures that there is no relative axial rotation between the positioning sleeve and the irregularly shaped elongated hole. Torque from the drive shaft 34 can be transmitted to the positioning sleeve of the manipulation shaft 31 through the irregularly shaped elongated hole. The transmission cavity section 343 has a transmission guide face 344, and the detent block 37 is internally provided with a gear guide face 374 that matches the transmission guide face 344. The curvatures of the transmission guide face 344 and the guide face on the inner bore of the detent block 37 are the same, or both are flat surfaces, allowing torque to be transmitted through this mating surface. The inner bore of the detent block 37 is sleeved over the transmission cavity section 343 with a clearance fit, enabling the detent block 37 to move axially on the transmission cavity section 343. When the detent block 37 is in the first and second positions, the gear guide face 374 is in contact with the transmission guide face 344 to restrict the movement of the drive shaft 34 relative to the detent block 37. Correspondingly, the main structure of the knob 35 is a rotating body that contains a cylindrical inner cavity. At least one end of the knob 35 has an opening, and the inner cavity is provided with an internal thread 353 of the knob. One end of the knob 35 can be axially nested with the detent block 37, and at the nesting position, there are at least one pair of mutually coupled "key features" and "keyway features" to ensure that after nesting, i.e., in the first position, the axial rotational freedom between them is restricted, and they only have mutual axial movement freedom. The proximal end of the drive shaft 34 is provided with a threaded connection portion, which comprises connecting threads and a threaded block 345. The internal thread 353 of the knob mates with the external thread of the threaded block 345. This threaded feature is configured to transmit motion and is preferably a trapezoidal thread. The outer side of the middle section of the main body of the knob 35 is provided with a recessed annular groove feature 354. The recessed annular groove of the recessed annular groove feature 354 is coaxial with the inner cavity, and the inner diameter of the recessed annular groove feature 354 is the same as the diameter of the knob annular groove 115 of the outer housing 11, with a clearance fit. The width dimension between the two side end faces of the recessed annular groove feature 354 is equal to the width dimension between the opposing side end faces of the two knob annular grooves 115 in the drive cavity of the outer housing 11, with a clearance fit. Similarly, in some implementations, the recessed annular groove feature 354 of the knob 35 can be replaced with a flange, wherein the cross-sections on both sides of the flange have the same width dimension as the opposing side end faces between the two knob annular grooves 115 on the outer housing 11, serving the same purpose. The other end of the knob 35 is provided with a handwheel 352 feature. The outer wall of the handwheel 352 is provided with some protruding or recessed pattern features to increase surface friction.

A conductive face 3431 may be provided at the end position of the transmission cavity section 343, and the cross-sectional area of the conductive face 3431 section is smaller than that of the transmission cavity section 343. The curvature of the conductive face 3431 is the same as the curvature of at least one inner side surface cross-section of the irregularly shaped hole 3451 of the threaded block, or both are flat surfaces. In some implementations, the transmission cavity section 343 is provided with connecting threads near its end. In some implementations, the threaded block 345 may directly serve as a feature on the drive shaft 34 or be fixedly connected to the drive shaft 34 as a separate component. The main structure of the threaded block 345 is a cylinder that comprises external thread features and mates with the internal thread 353 of the knob 35 in its inner cavity. When the threaded block 345 is a separate component, it has an inner bore in the middle of the cylinder, and the central axis of the inner bore coincides with the axis of the cylinder. In this example, the threaded block 345 with the irregularly shaped hole 3451 can be sleeved over the conductive face 3431 at the end of the transmission cavity section. Since the cross-sectional area at the conductive face 3431 is smaller than the main cross-sectional area of the transmission cavity section 343, the threaded block 345 will be restricted from axial displacement in one direction. Then, a lock nut 346 is screwed onto the connecting threads to completely restrict the axial displacement of the threaded block 345, thereby firmly fixing and connecting the threaded block 345 to the drive shaft 34. Compared to the solution of directly bonding or welding the threaded block 345 onto the drive shaft 34, the solution disclosed in this example has the advantages of being detachable and easy to assemble.

During the opening and closing process of the closure members 52, the fluid injection cavity section 342 remains within the fluid injection cavity 101 of the outer housing 11, and the transmission cavity section 343 is located within the drive cavity of the outer housing 11. The drive shaft 34 comprises a control shaft connection end 341 at the distal end, which is provided with an irregularly shaped elongated hole that matches the positioning sleeve of the manipulation shaft 31. Torque from the drive shaft 34 can be transmitted to the positioning sleeve of the manipulation shaft 31 through the irregularly shaped elongated hole, enabling the drive shaft 34 to drive the rotation of the manipulation shaft 31 and thus realizing the opening and closing control of the distal closure members 52.

In this embodiment, one of the first snap-fit structure 371 and the second snap-fit structure 351 is a key structure, and the other is a keyway structure. The key structure and the keyway structure are coupled in the first position to restrict the rotation of the knob 35 relative to the detent block 37. The switching mechanism also comprises a clutch block 36 and a spring 362. The outer housing 11 is provided with a gear block annular groove 114 that restricts the rotation of the detent block 37 and guides the axial sliding of the detent block 37 relative to the outer housing 11. The proximal end of the clutch block 36 is provided with a first compression structure 361, and the distal end of the detent block 37 is provided with a second compression structure 372. The spring 362 provides a biasing force to the clutch block 36, causing the first compression structure 361 to press against the second compression structure 372. The first compression structure 361 and the second compression structure 372 are configured such that when the detent block 37 rotates, the second compression structure 372 transmits a biasing force that drives the clutch block 36 to move distally to the first compression structure 361. Therefore, when the detent block 37 is in the first position and the knob 35 drives the drive shaft 34, due to the assembly relationship between the first compression structure 361 and the second compression structure 372, the clutch block 36 is continuously pressed and moved distally during rotation and then reset by the spring 362. This creates a clicking sensation during rotation, making the adjustment process more stable. In some implementations, the second compression structure 372 is a protruding tooth, and the first compression structure 361 is a mating engaging tooth that matches the protruding tooth.

In some implementations, the switching mechanism also comprises a toggle plate 38 that is axially fixed to the detent block 37. In this example, the outer housing 11 is provided with a toggle plate guide rail located between the clutch block annular groove 113 and the sliding groove of the knob 35. The top surface of the toggle plate guide rail mates with the positive guide face of the toggle plate 38, and the side surface of the toggle plate guide rail mates with the lateral guide face of the toggle plate 38, allowing the toggle plate 38 to only move axially and restricting its axial rotation. The toggle plate 38 is provided with a first snap 381, and the outer housing 11 is provided with a second snap 116 that matches the first snap 381. When the toggle plate 38 moves the detent block 37 to the second position, the first snap 381 engages with the second snap 116 to restrict the detent block 37 from moving back to the first position. When the detent block 37 is maintained in the second position, rotating the knob 35 will cause it to rotate relative to the drive shaft 34. Due to the threaded engagement, the threaded block 345 at the distal end of the drive shaft 34 moves proximally, driving the drive shaft 34 to slide proximally, which in turn drives the manipulation shaft 31 to slide together, thereby achieving separation from the distal valve clip device 5. In some implementations, a sliding button 39 is also slidably provided on the exterior of the outer housing 11. The sliding button 39 passes through the outer housing 11 and is connected to the toggle plate 38 to drive the toggle plate 38 to slide, facilitating the switching process by simply sliding the sliding button 39.

Furthermore, the moving seat 13 comprises an inner cavity 131. The control shaft connection end 341 is located within the inner cavity 131 of the moving seat and is capable of rotating relative to the moving seat 13. In some implementations, the length of the control shaft connection end 341 matches the length of the inner cavity 131 of the moving seat. The main structure of the moving seat 13 is a box-like structure, which, in this example, is a rectangular box. The surfaces of the moving seat 13 parallel to the reference axis serve as guide faces, divided into an inner guide face 132 and an outer guide face 133 based on their interior and exterior positions. The surfaces perpendicular to the reference axis are end faces, divided into an inner end face 1311 and an outer end face based on their interior and exterior positions. On the thin walls of the box on both sides, between the inner and outer end faces, a manipulation shaft annular groove 134 and a drive shaft annular groove 135 are provided, respectively. These two annular grooves are coaxial and, when assembled with the outer housing 11, are coaxial with the reference axis. The diameter of the manipulation shaft annular groove 134 is preferably equal to the outer diameter of the manipulation shaft 31. The diameter of the drive shaft annular groove 135 is preferably slightly larger than the diameter of the fluid injection cavity section 342 of the drive shaft 34 to ensure smooth rotation of the drive shaft 34 within the annular groove, while the diameter of the annular groove is smaller than the minimum radial dimension of the indicating part 4, thereby preventing the indicating part 4 from disengaging from the interior.

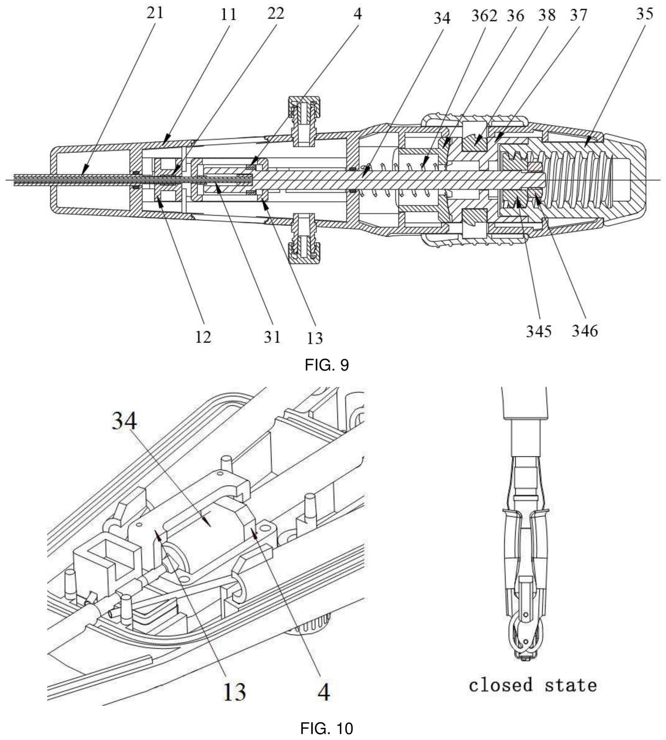

The indicating part 4 is provided with threaded features and is threadedly engaged with the control shaft connection end 341. The outer surface of the indicating part 4 is slidably connected to the inner guide face 132 and is relatively fixed in the rotational direction. In some implementations, the indicating part 4 is provided with a threaded hole, and the threaded hole of the indicating part 4 is threadedly connected to the peripheral threads of the control shaft connection end 341. In some implementations, the outer surface of the indicating part 4 may be quadrilateral to prevent relative rotation with respect to the moving seat 13. Therefore, when the drive shaft 34 rotates, the control shaft connection end 341 of the drive shaft 34 drives the rotation of the manipulation shaft 31 while also rotating relative to the threaded hole of the indicating part 4. Consequently, the indicating part 4 undergoes axial displacement relative to the drive shaft 34 and slides along the inner guide face. Thus, the position of the indicating part 4 relative to the moving seat 13 can accurately and intuitively reflect the number of rotations of the drive shaft 34, which corresponds to the opening angle of the distal closure member 52. For specific details, please refer to Figures 10-14 in the accompanying drawings. Further preferably, in this embodiment, to enable intuitive observation of the movement extent of the indicating part, the moving seat 13 is provided with a first observation window 136 for observing the indicating part 4. The outer housing 11 is provided with a second observation window 117 corresponding to the first observation window 136 to facilitate observation by the operator. In a further preferred design, position markings or angle markings corresponding to the current state of the closure member 52 can be provided on the second observation window 117 for more intuitive display. In this embodiment, the inner cavity 131 of the moving seat comprises an inner end face 1311. The inner end face 1311 cooperates with both ends of the control shaft connection end 341 to restrict the control shaft connection end 341 within the inner cavity 131 of the moving seat. Therefore, when the indicating part 4 moves to abut against the inner end face 1311, it cannot move further, thereby restricting the rotation of the drive shaft 34 and preventing excessive rotation of the drive shaft from driving the manipulation shaft 31, thus protecting the manipulation shaft and the distal closure member.

In this embodiment, leveraging the rotational characteristic of the drive shaft and the special transmission relationship between the indicating part and the moving seat, the operator can intuitively understand the degree of opening and closing of the closure member of the valve clip device at the distal end without the need for additional in-vivo imaging equipment for observation. This also addresses the technical issue where improper operation might lead to excessive rotational angles, causing the closure member to exceed the limit position of deployment and potentially damaging the device.

In this example, a manipulation shaft positioning sleeve 32 is fixedly fitted over the proximal end of the manipulation shaft 31. The control shaft connection end 341 comprises a specially shaped elongated hole, and the outer surface of the manipulation shaft positioning sleeve 32 matches the inner surface of the specially shaped elongated hole to restrict relative rotation between the manipulation shaft 31 and the drive shaft 34. The manipulation shaft positioning sleeve 32 and the specially shaped elongated hole are configured to slide axially relative to each other, with the length of the specially shaped elongated hole being greater than that of the manipulation shaft positioning sleeve 32. The inner diameter of the manipulation shaft annular groove 134 is smaller than the maximum outer diameter of the manipulation shaft positioning sleeve 32. In some implementations, the manipulation shaft positioning sleeve 32 can be directly fixedly connected to the manipulation shaft 31, with the manipulation shaft positioning sleeve 32 on the manipulation shaft 31 extending into the specially shaped elongated hole of the control shaft connection end 341 of the drive shaft 34. The cross-section of the specially shaped elongated hole may or may not be the same as the shape of the outer axial cross-section of the manipulation shaft positioning sleeve 32, as long as it ensures that there is no relative axial rotation between the manipulation shaft positioning sleeve 32 and the specially shaped elongated hole. The specially shaped elongated hole and the manipulation shaft positioning sleeve 32 are in clearance fit, allowing the manipulation shaft positioning sleeve 32 to slide axially therein, which is referred to as a floating connection. When the drive shaft 34 at the handle end rotates, the manipulation shaft positioning sleeve 32 extending into the specially shaped elongated hole is driven, which in turn drives the rotation of the manipulation shaft 31, followed by the rotation of the control rod on the closure member 52. The control rod then drives the transmission rod on the closure member 52. Due to the helical transmission between the base and the transmission rod on the closure member 52, rotational motion is converted into linear motion, ultimately achieving the opening and closing motion of the closure member. Due to the transmission form between the base and the transmission rod, when the transmission rod rotates, its axial displacement also changes, leading to a tendency for the manipulation shaft 31 to undergo axial displacement relative to the inner tube 21. Since the drive shaft 34 only rotates relative to the outer housing 11 during the opening and closing of the closure member, while remaining axially fixed, this axial displacement tendency can be offset by axial stretching or compression of the manipulation shaft 31, or by its axial displacement. When the closure member 52 is released, the manipulation shaft 31 is pulled proximally by the drive shaft 34, causing the control rod to separate from the transmission rod. However, due to the limited axial travel of the drive shaft 34, the manipulation shaft 31 is required to have a certain degree of axial rigidity. If the axial elongation is too great, the drive shaft 34 may complete its full travel while the transmission rod and the control rod remain connected, as the travel is absorbed by the elongation of the flexible manipulation hose, thereby affecting the normal release of the closure member 52. Given the axial rigidity requirement of the manipulation shaft 31, the axial displacement tendency of the manipulation shaft 31 generated during the opening and closing of the closure member is more reliably offset by allowing the manipulation shaft 31 to move axially. The floating connection method in this example precisely meets the requirement that the axial displacement of the manipulation shaft 31 during the opening and closing of the closure member is not affected by the drive shaft 34, allowing the manipulation shaft 31 to possess axial rigidity. If the manipulation shaft 31 and the drive shaft 34 were fixedly connected, the manipulation shaft 31 would need to have a certain degree of axial elasticity to eliminate the axial tendency generated during the opening and closing of the closure member 52. However, the elongation caused by axial elasticity under tension is not conducive to the release of the closure member 52. Additionally, the floating connection also facilitates the assembly of the control handle and makes it easier for the operator to disassemble the control handle under special circumstances. Preferably, a floating elastic member 320, which can be a spring, is also provided between the proximal end of the manipulation shaft positioning sleeve 32 and the bottom surface of the specially shaped elongated hole. When the floating elastic member 320 is in its natural state, the distal end of the manipulation shaft positioning sleeve 32 is located within the specially shaped elongated hole. After the floating elastic member 320 is placed, a pre-compression force exists between the control rod and the transmission rod through the transmission of the manipulation shaft 31, preventing accidental release due to unexpected impacts.

In this embodiment, the capturing member 51 requires the manipulation wire 33 to achieve the release process, thereby cooperating with the closure member 52 to clamp the tissue. Specifically, to compactly and reasonably arrange the manipulation wire 33 and the manipulation shaft 31 while ensuring they do not interfere with each other, the inner tube assembly 2 in this embodiment comprises an inner tube 21 and a sleeve 24 for guiding the manipulation wire 33. The inner tube 21 comprises an inner layer 211 and an outer layer 212. The inner layer 211 is provided with a cavity 2111 for the manipulation shaft 31 to pass through, while the outer layer 212 is provided with a channel 215 for the manipulation wire 33 to pass through. The proximal end of the inner layer 211 extends beyond the outer layer 212, and at least a portion of the extending section is fixedly fitted with an inner tube positioning sleeve 22. Correspondingly, the housing assembly 1 further comprises a fixed seat 12 disposed within the outer housing 11. The fixed seat 12 comprises a positioning sleeve slot 121 and sleeve guide slots 122 located on both sides of the positioning sleeve slot 121, with the two sleeve guide slots 122 tapering from the proximal end to the distal end. The positioning sleeve slot 121 matches the inner tube positioning sleeve 22, and the sleeve guide slots 122 match the sleeve 24. The distal end of the fixed seat 12 is also provided with an annular groove 123 for the inner layer 211 to pass through. The annular groove 123 communicates with the positioning sleeve slot 121, and its inner diameter is larger than that of the inner layer 211 but smaller than that of the inner tube positioning sleeve 22.

The fixed seat 12 is provided with a positioning sleeve slot 121 and an annular groove 123. The cross-sectional characteristics of the positioning sleeve slot 121 are consistent with those of the inner tube positioning sleeve 22. Preferably, the cross-sectional dimensions and length of the positioning sleeve slot 121 are consistent with those of the inner tube positioning sleeve 22. Both ends of the positioning sleeve slot 121 in the length direction are provided with annular grooves 123. The diameter of the annular groove 123 is larger than that of the inner layer 211 of the inner tube 21, and its area is smaller than the cross-sectional area of the positioning sleeve slot 121. The sleeve guide slots 122 are disposed on both sides of the positioning sleeve slot 121, symmetrically distributed in a "V" shape on both sides of the positioning sleeve slot 121. The sleeve guide slots 122 are configured to guide the sleeve 24, aligning the port of the sleeve 24 with the cavity port of the manipulation wire 33 in the inner tube 21, and fixedly connecting the sleeve 24 to the fixed seat 12. In this example, the fixed seat 12 has a rectangular block structure, with the positioning sleeve slot 121 and sleeve guide slots 122 provided on one of its surfaces. During use, the slots of two fixed seats 12 are aligned to form an integral whole. The split design of the fixed seat 12 facilitates assembly and disassembly. In some implementations, the fixed seat 12 is disposed within the fluid injection cavity, with the central axis of the positioning sleeve slot 121 of the fixed seat 12 coaxial with the reference axis. In some implementations, when the fixed seat 12 is an independent component, a slot for the fixed seat 12 is provided within the fluid injection cavity, allowing the fixed seat 12 to be inlaid within it. After inlaying, it is ensured that the central axis of the positioning sleeve slot 121 on the fixed seat 12 is coaxial with the reference axis.

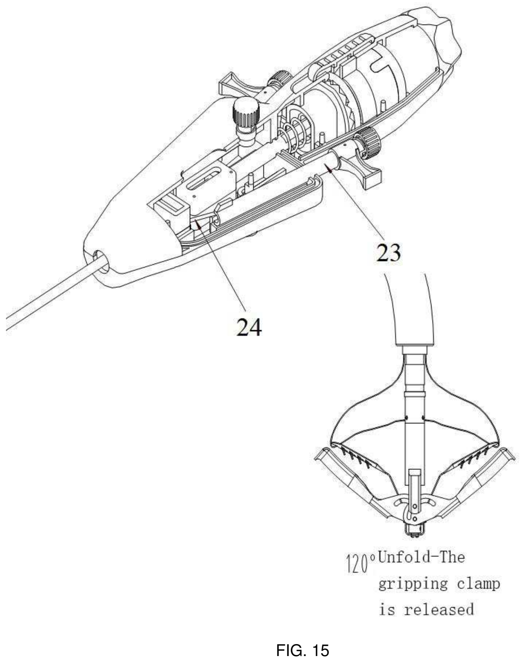

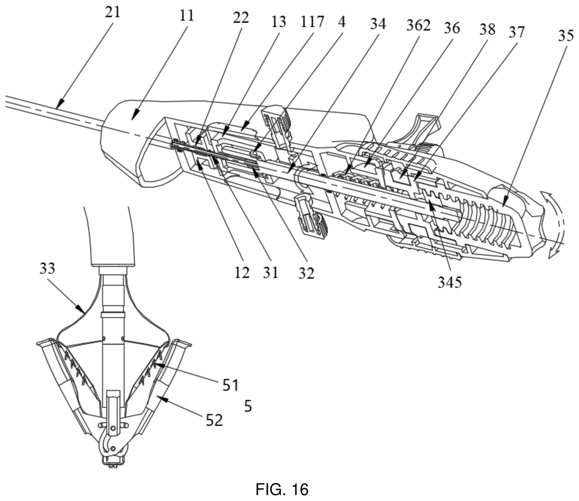

Within the fluid injection cavity of the outer housing 11, there is also a guide rail 112. The orientation of the guide rail 112 is approximately from the push-pull rod annular groove to the chute of the inner tube 21. The control assembly 3 further comprises a push-pull rod 23 slidably disposed on the guide rail 112, with the push-pull rod 23 being fixedly connected to the distal end of the manipulation wire 33. The control assembly 3 also comprises a support tube 25 disposed within the inner cavity of the push-pull rod 23, with the support tube 25 being sleeved over the sleeve 24. The release of the manipulation wire 33 can be achieved through the movement of the push-pull rod 23, as shown in Figures 15 and 16. In this example, the guide rail 112 consists of two parallel rib grooves, with the spacing between the two parallel ribs equal to the width of the convex rib on the guiding head of the push-pull rod 23, forming a clearance fit. In another example, the guide rail is a single rib, with the width of the guide rail equal to the width of the slot on the guiding head.

Based on the structure of this embodiment, the specific process for clamping and fixing the valve clip device onto the heart is as follows:
delivering the valve clip device to the designated position within the heart;
rotating the knob to drive the synchronous rotation of the manipulation shaft, thereby controlling the opening angle of the closure member;
after the closure member contacts the valve, controlling the push-pull rod to manipulate the manipulation wire, achieving the release of the capturing member. The capturing member, in cooperation with the closure member, clamps the tissue;
further rotating the knob to drive the closure member, which is clamping the tissue, to a predetermined angle;
pushing the toggle button to enable the switching mechanism to switch the knob to a state where it rotates relative to the drive shaft;
continue rotating the knob, wherein under the cooperation of the internal thread of the knob, the threaded block moves proximally to drive the drive shaft to slide proximally; and
the drive shaft causing the manipulation shaft to disengage from the valve clip device, leaving it inside the human body.

The embodiment of this application also provides a delivery system for an interventional device, specifically designed to transport a valve clip device to a designated location within the heart. Subsequently, through a control system, the valve clip device is manipulated to clamp and secure the damaged valve tissue, after which it remains in the heart as an implant. The delivery system is then detached from the valve clip device, leaving the latter inside the human body before being withdrawn externally.

In the treatment of patients with mitral regurgitation, the delivery system can access the body through the femoral vein, passing through the inferior vena cava, right atrium, interatrial septum, and left atrium to reach the mitral valve. Under the guidance of three-dimensional echocardiography and digital subtraction angiography, the valve clip device is controlled to grasp the anterior and posterior leaflets of the mitral valve. The anterior and posterior leaflets of the mitral valve are clamped by the valve clip device, transforming the mitral valve from a large single orifice during systole into a smaller dual orifice, achieving "edge-to-edge" repair and thereby reducing mitral regurgitation. In the treatment of patients with tricuspid regurgitation, the valve clip device is controlled via the delivery system to capture and clamp the tricuspid valve leaflets, thus reducing tricuspid regurgitation.

It should be noted that the valve clip device 5 comprises a capturing member 51 and a closure member 52, which work in conjunction to clamp the valve tissue.

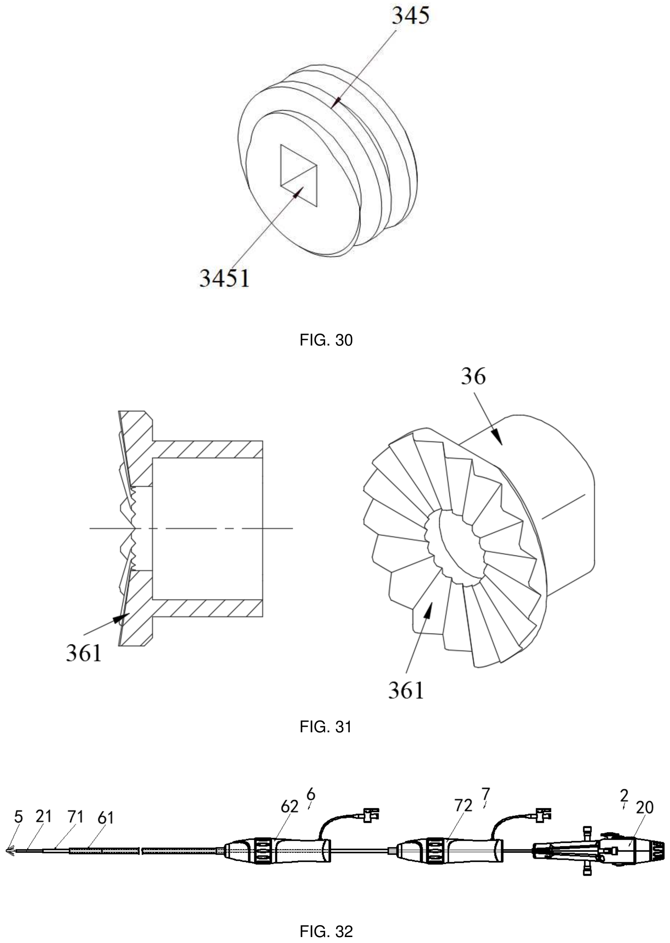

As illustrated in Figures 32-49, the delivery system of this application comprises a control system, a guiding catheter assembly, a middle tube assembly 7, an inner tube assembly 2, and a manipulation shaft 31. The guiding catheter assembly comprises an outer tube assembly 6, which further comprises an outer tube body 61. Within the outer tube body 61, an outer tube deflection member 611 is disposed, designed to control the deflection of at least a portion of the outer tube body 61.

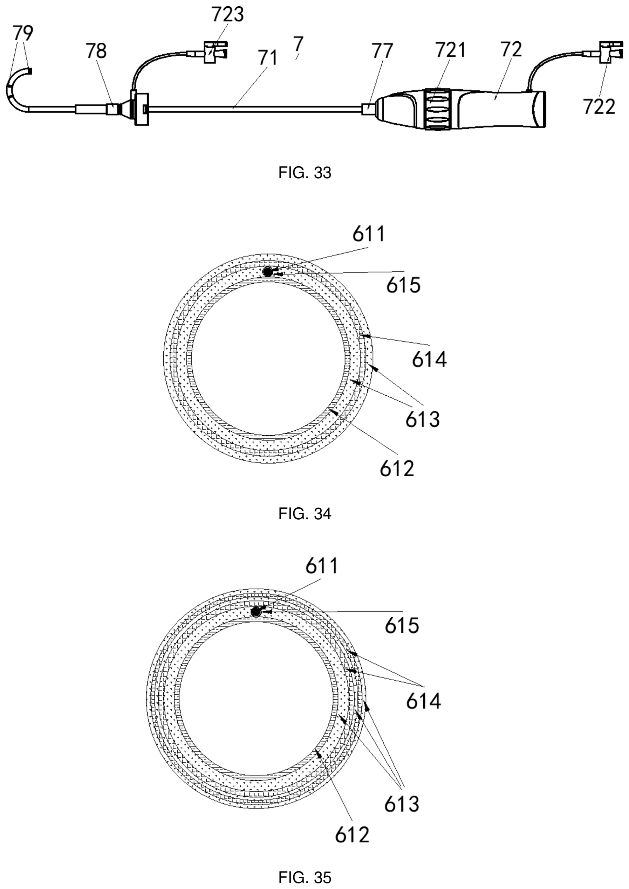

As shown in Figures 33-35, the outer tube assembly 6 consists of an outer tube body 61 and an outer tube handle 62. The outer tube body 61 is radially structured from the inside out with an inner lining layer 612 and an outer tube body coating layer 613. Embedded within the outer tube body coating layer 613 are at least one layer of outer tube support layers 614, spaced apart. Additionally, at least one outer tube deflection member liner tube 615 is disposed within the outer tube body coating layer 613, housing the outer tube deflection member 611. In this embodiment, the outer tube body 61 is radially structured from the inside out with an inner lining layer 612 followed by an outer tube body coating layer 613, within which two spaced-apart outer tube support layers 614 are embedded. Specifically, the outer tube deflection member 611 can be a deflection wire, which may be made of metal. By pulling the deflection wire, the distal end of the outer tube body 61 can be bent and deformed. The inner lining layer 612 can be fabricated from a polymer material with good lubricating properties, such as polytetrafluoroethylene (PTFE), to enhance the lubricity of the inner wall of the cavity. The outer tube body coating layer 613 can be a tubular layer made of, but not limited to, block polyetheramide, nylon, or other polymer materials. Different materials or materials with varying hardness can be selected for different hardness segments of the tube body. In some implementations, the outer tube support layers 614 can be fabricated from flat wires or round wires in a diamond-shaped braiding pattern, or they can be wound springs, or etched metal tubes. In other implementations, the outer tube support layers 614 can be single-layered or multi-layered, or they can be a combination of braided layers and spring layers. By adjusting the braiding density of the outer tube support layers 614 in different segments of the tube body, the flexibility and stiffness of each segment can be altered. In this embodiment, the outer tube support layers 614 serve to increase the flexibility and radial support force of the outer tube body 61, preventing it from bending easily.

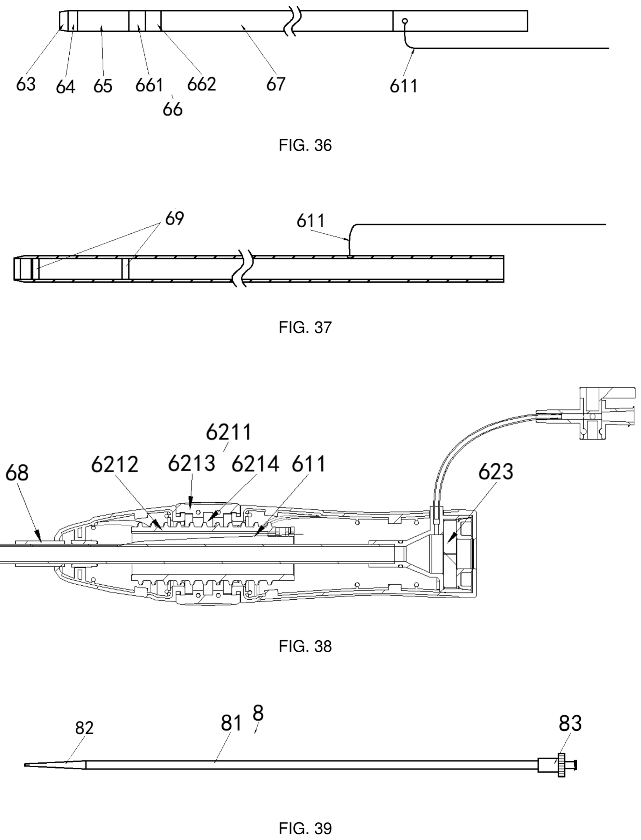

In some embodiments, as illustrated in Figures 36 and 37, the outer tube body 61 comprises, from the distal end to the proximal end, a deflection tip 63, a pull-ring section 64, a deflection section 65, and a main body section 67. The pull-ring section 64 is connected to the distal end of the outer tube deflection member 611, while the proximal end of the outer tube deflection member 611 is connected to the outer tube handle 62. The stiffness of the deflection section 65 is less than that of the main body section 67. Preferably, in other possible embodiments, at least one transition section 66 is disposed between the deflection section 65 and the main body section 67, with the stiffness of the transition section 66 being greater than that of the deflection section 65 but less than that of the main body section 67. In this embodiment, the outer tube body 61 comprises, in sequence from the distal end to the proximal end, a deflection tip 63, a pull-ring section 64, a deflection section 65, a first transition section 661, a second transition section 662, and a main body section 67. The stiffness increases sequentially from the deflection section 65, through the first transition section 661 and the second transition section 662, to the main body section 67. In this embodiment, the distal end of the outer tube deflection member 611 is connected to the pull-ring section 64, and the stiffness of the outer tube body 61 increases sequentially from the distal end to the proximal end. By pulling the outer tube deflection member 611, the curvature of the outer tube body 61 can be adjusted.

Preferably, in some embodiments, the outer tube body 61 is also provided with at least one outer tube radiopaque marker 69. The outer tube radiopaque marker 69 is visible under X-ray, facilitating the intraoperative confirmation of the position of the outer tube body 61 within the body. In some embodiments, the outer tube radiopaque marker 69 is disposed on the deflection tip 63 of the outer tube body 61 and can be a tubular segment with platinum material added.

As shown in Figure 38, the outer tube handle 62 is connected to the proximal end of the outer tube body 61. The outer tube handle 62 is equipped with an outer tube deflection assembly 621 for adjusting the curvature of the outer tube body 61. The outer tube deflection assembly 621 comprises an outer tube knob 6211 and an outer tube threaded member 6212. The outer tube knob 6211 comprises a knob fixing sleeve 6213 and an internally threaded member 6214. The knob fixing sleeve 6213 is securely fitted over the externally threaded member 6214, with the internal threads of the internally threaded member 6214 mating with the external threads of the outer tube threaded member 6212. In some embodiments, both the knob fixing sleeve 6213 and the internally threaded member 6214 are installed in a split manner, with the split joint of the knob fixing sleeve 6213 and that of the internally threaded member 6214 being oriented at a 90° angle to each other. This design eliminates the need for fasteners such as screws during the assembly of the entire outer tube handle, making the assembly process more convenient. The outer tube threaded member 6212 is sleeved over the outer tube body 61 and is connected to the outer tube deflection member 611. The outer tube knob 6211 is designed to rotate and drive the axial movement of the outer tube threaded member 6212, thereby pulling the outer tube deflection member 611. Specifically, in some embodiments, the outer tube threaded member 6212 can be a threaded tube with external threads, and the outer tube knob 6211 is threadedly connected to the outer tube threaded member 6212 via the internally threaded member 6214. By rotating the outer tube knob 6211 on the outer tube handle 62, the threaded configuration enables the outer tube knob 6211 to move the outer tube threaded member 6212 axially back and forth, thus pulling the outer tube deflection member 611 connected to the outer tube threaded member 6212 to control the deflection of the outer tube body 61.

Preferably, in some embodiments, a stress diffusion tube 68 for the outer tube is also provided at the junction between the outer tube handle 62 and the outer tube body 61. The stress diffusion tube 68 is designed to prevent stress concentration at the junction of the two components, which could otherwise lead to easy bending. The outer tube handle 62 is also equipped with an outer tube Luer three-way valve 622, which is used for connecting to a syringe to facilitate flushing of the outer tube assembly 6 before and after use. **In** some embodiments, an outer tube hemostasis valve 623 is also provided within the outer tube handle 62, positioned at the distal end of the outer tube handle 62. The outer tube hemostasis valve 623 is designed to selectively seal the cavity of the outer tube body 61 to minimize blood loss and prevent air from entering the delivery system and forming an air embolism. **In** practical applications, when the middle tube assembly or a guide catheter passes through the outer tube handle 62, particularly through the outer tube hemostasis valve, the cavity of the outer tube handle can be sealed by the outer tube hemostasis valve. Similarly, when the middle tube assembly or guide catheter is removed or not extending through the outer tube handle, the inner cavity of the outer tube handle can also be sealed by the outer tube hemostasis valve.

Specifically, the guiding catheter assembly further comprises a guide catheter 8, which is detachably positioned within the outer tube body 61. If the delivery system of this embodiment uses a guidewire to access the heart, the guidewire can first be advanced through the blood vessel to the designated position within the heart. Subsequently, the guide catheter 8 is mated with the outer tube assembly 6 and advanced along the guidewire to reach the heart, establishing a delivery pathway. After the delivery pathway is established, the guidewire and the guide catheter 8 are withdrawn. Then, the middle tube assembly 7, in conjunction with the inner tube assembly 2, is configured to deliver the valve clip device 5 to the designated position within the heart through the cavity of the outer tube assembly 6. Specifically, the inner diameter of the guide catheter 8 is smaller than that of the outer tube body 61. By placing the guidewire within the smaller-diameter guide catheter 8, better compliance and guidance are achieved. As illustrated in Figure 39, in some embodiments, the guide catheter 8 comprises a main body portion 81 and a guiding head 82 that are interconnected. The outer diameter of the guiding head 82 gradually decreases from the end connected to the main body portion 81 to the end distal from it. The configuration of the guiding head 82 enhances the compliance and guidance when the outer tube assembly 6 and the guide catheter 8 are cooperatively inserted into the blood vessel, thereby preventing damage to the vascular wall. Preferably, in some possible embodiments, the guiding head 82 can be a conical head. The stiffness of the guiding head 82 is less than or equal to that of the main body portion 81. Specifically, the guide catheter 8 further comprises a Luer head 83, which is positioned at the end of the main body portion 81 distal from the guiding head 82.

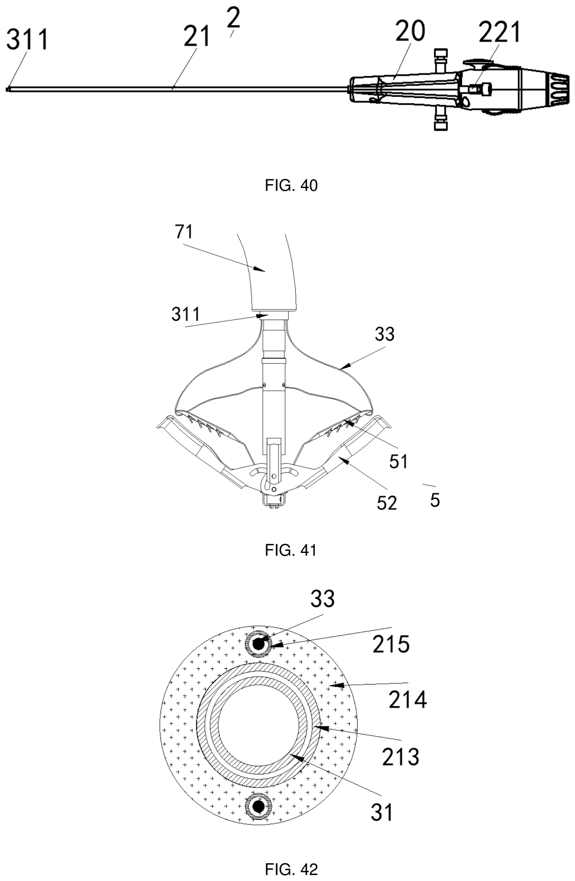

As shown in Figures 40 and 41, the inner tube assembly 2 comprises an inner tube 21, through which a manipulation wire 33 is threaded. The manipulation wire 33 is connected to the capturing member 51 of the valve clip device 5 and is configured to control the capturing action of the capturing member 51. Specifically, the inner tube assembly 2 further comprises a control handle 20, which is connected to the inner tube 21. The control handle 20 is equipped with a joystick 221 that is connected to the manipulation wire 33. In some embodiments, the joystick 221 is slidably or rotatably connected to the control handle 20. By pushing or rotating the joystick 221, the movement of the manipulation wire 33 can be controlled, thereby opening and closing the capturing member 51 of the valve clip device. In some embodiments, the manipulation wire 33 can be made of metal. In other possible embodiments, the front segment of the manipulation wire 33 can be made of a polymer material, while the rear segment is made of metal. The polymer material ensures the flexibility of the front end of the manipulation wire 33, while the metal material guarantees its pushability. In some embodiments, the manipulation wire 33 can be a braided body consisting of a single strand or multiple strands of wire.

As shown in Figures 42-44, in some embodiments, the inner tube 21 is radially structured from the inside out with an inner layer 211 and an outer layer 212. At least one channel 215 is disposed within the outer layer 212, and the manipulation wire 33 is positioned within at least one of these channels 215. In other possible embodiments, the inner tube 21 is radially structured from the inside out with an inner layer 211, a reinforcing tube layer 218, and an elastic tube layer 219 in sequence. At least one channel 215 is disposed within the elastic tube layer 219, and the manipulation wire 33 is positioned within at least one of these channels 215.

In some embodiments, the inner layer 211 can be fabricated by engraving stainless steel or nickel-titanium alloy materials. The engraved patterns allow for the adjustment of the tube's stiffness and flexibility, enabling it to possess good torque controllability while maintaining a soft foundation. In other embodiments, the inner layer 211 can also be a single-layer or multi-layer spring tube with torque controllability. The outer layer 212 can be a coating made of high-molecular materials such as block polyetheramide resin or thermoplastic polyurethane elastomer, which can be applied to the inner layer 211 using hot-melt technology. The outer layer 212 serves to secure the channels 215 and enhance the flexibility and post-bending straightening capability of the inner tube 21. The channels 215 provide a cavity for the axial movement of the manipulation wire 33. The channels 215 can be made of at least one material with good lubricity and ease of thin-wall fabrication, such as polytetrafluoroethylene (PTFE), polyetheretherketone (PEEK), or polyimide. In some possible embodiments, the number of channels 215 can be one or multiple. Preferably, in this embodiment, the number of channels 215 can be two or four. The reinforcing tube layer 218 can be a layer of polyethylene terephthalate (PET) heat-shrunk onto the outer surface of the inner layer 211. The elastic tube layer 219 can be a layer of block polyetheramide resin tube coated over the reinforcing tube layer 218. In this embodiment, the inner tube 21, comprising multiple layers, exhibits certain flexibility, good post-bending straightening capability, and excellent torque controllability.

Specifically, the manipulation shaft 31 is inserted into the cavity 2111 of the inner tube 21. The distal end of the manipulation shaft 31 is equipped with a connecting portion 311, which is detachably connected to the valve clip device 5. The manipulation shaft 31 is capable of rotating relative to the inner tube 21 to control the opening and closing of the closure member 52 of the valve clip device 5. The proximal end of the manipulation shaft 31 is connected to the control handle 20, through which the rotation of the manipulation shaft 31 relative to the inner layer 211 can be controlled. Radially from the inside out, the manipulation shaft 31 is sequentially composed of a spring tube layer 312, a manipulation main body tube 313, and a heat-shrink tube layer 314. In some embodiments, the manipulation main body tube 313 can be a metal engraved tube. Preferably, the manipulation main body tube 313 can be engraved from stainless steel or nickel-titanium alloy materials. The engraved patterns allow for the adjustment of the tube's stiffness and flexibility, enabling it to possess good torque controllability while maintaining a soft foundation. The spring tube layer 312 is a tube with a helical structure formed by winding wire/filament materials. The heat-shrink tube layer 314 can be a layer of polytetrafluoroethylene (PTFE) heat-shrunk onto the outer surface of the manipulation main body tube 313. In this embodiment, the manipulation shaft 31, in cooperation with the inner tube assembly 2, delivers the valve clip device 5 to the designated position. By controlling the rotation of the manipulation shaft 31, the opening and closing of the closure member 52 are controlled, while the capturing member 51 is controlled by the manipulation wire 33 for capturing. After the valve clip device completes the grasping and repair of the heart valve, the manipulation shaft 31 is controlled to completely separate the connecting portion 311 and the manipulation wire 33 from the valve clip device.

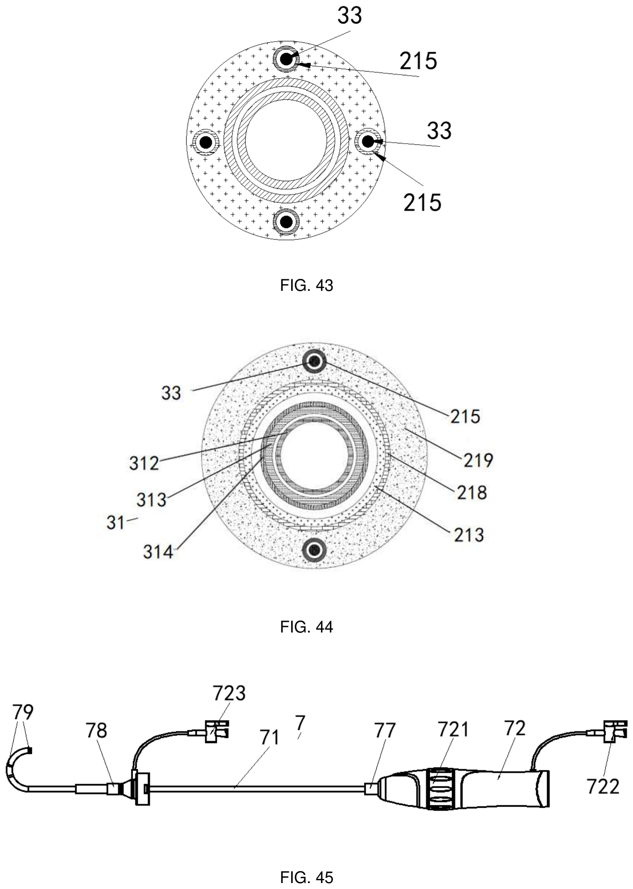

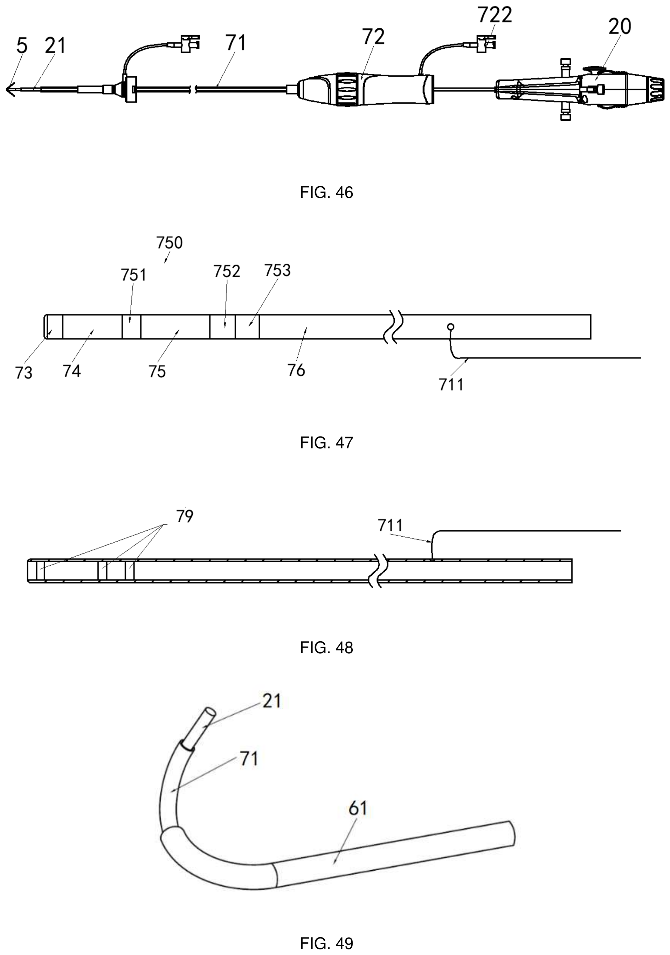

As shown in Figures 45 and 46, the middle tube assembly 7 comprises a middle tube body 71 and a middle tube handle 72. A middle tube deflection member 711 is disposed within the middle tube body 71, which is configured to control the deflection of at least a portion of the middle tube body 71. The middle tube body 71 is capable of accommodating the inner tube 21, while the outer tube body 61 is capable of accommodating the middle tube body 71. Specifically, in this embodiment, the inner diameter of the outer tube body 61 is greater than the outer diameter of the middle tube body 71, and the inner diameter of the middle tube body 71 is greater than the outer diameter of the inner tube 21. The combined assembly of the middle tube assembly 7 and the inner tube assembly 2 in this embodiment enters the heart through the delivery pathway established by the outer tube assembly 6. During this process, the middle tube deflection member 711 can be configured to control the deflection of the middle tube body 71, enabling the inner tube assembly 2 to smoothly reach the designated position within the heart. The middle tube deflection member 711 can be a deflection wire, which can be made of metal. By pulling the deflection wire, the distal end of the middle tube body 71 can be bent and deformed.

In some embodiments, the middle tube body 71 is radially structured with a middle tube inner liner layer and a middle tube body coating layer. At least one middle tube support layer is embedded within the middle tube body coating layer. At least one middle tube deflection component liner is disposed within the middle tube body coating layer, and the middle tube deflection member 711 is positioned within the middle tube deflection component liner. Specifically, the middle tube inner liner layer can be made of a high-molecular material with good lubricity, such as polytetrafluoroethylene (PTFE), to increase the lubricity of the inner cavity wall. The middle tube body coating layer can be a tube layer made of, but not limited to, high-molecular materials such as block polyetheramide or nylon. Different materials and/or materials with varying hardness can be selected for different hardness segments of the tube body. In some embodiments, the middle tube support layer can be fabricated using flat wires or round wires in a diamond-shaped braiding pattern, or it can be made by winding wire/filament materials into a spring form. In other embodiments, the middle tube support layer can be single-layer or multi-layer, or it can be a combination of a braided layer and a spring layer. By adjusting the braiding density of the middle tube support layer in different segments of the tube body, the stiffness and flexibility of each segment can be altered. In this embodiment, the middle tube support layer is configured to enhance the flexibility, torque controllability, and radial support force of the middle tube body 71, preventing it from easily bending and ensuring good torque controllability.

As shown in Figure 47, in some embodiments, the middle tube body 71 comprises, from the distal end to the proximal end, a middle tube pull-ring section 73, a middle tube deflection section 74, a middle tube compliant section 75, and a middle tube main body section 76. The middle tube pull-ring section 73 is connected to the distal end of the middle tube deflection member 711, while the proximal end of the middle tube deflection member 711 is connected to the middle tube handle 72. The stiffness of the middle tube deflection section 74 is less than that of the middle tube compliant section 75, and the stiffness of the middle tube compliant section 75 is less than that of the middle tube main body section 76.

Preferably, in other possible embodiments, at least one middle tube transition section 750 is provided between the middle tube main body section 76 and the middle tube compliant section 75, as well as between the middle tube compliant section 75 and the middle tube deflection section 74. The stiffness of the middle tube transition section 750 is less than that of the middle tube main body section 76. Preferably, in this embodiment, the middle tube body 71 comprises, from the distal end to the proximal end in sequence, the middle tube pull-ring section 73, the middle tube deflection section 74, a first middle tube transition section 751, the middle tube compliant section 75, a second middle tube transition section 752, a third middle tube transition section 753, and the middle tube main body section 76. The stiffness of the middle tube pull-ring section 73, the middle tube deflection section 74, the first middle tube transition section 751, and the middle tube compliant section 75 increases in sequence. Similarly, the stiffness of the second middle tube transition section 752, the third middle tube transition section 753, and the middle tube main body section 76 also increases in sequence. In this embodiment, the stiffness of the middle tube body 71 increases from the distal end to the proximal end. The distal end of the middle tube deflection member 711 is connected to the middle tube pull-ring section 73, and by pulling the middle tube deflection member 711, the curvature of the middle tube body 71 can be adjusted.

Preferably, as shown in Figure 48, in some embodiments, the middle tube body 71 is further equipped with at least one middle tube radiopaque marker 79. The middle tube radiopaque marker 79 is visible under X-ray, facilitating the confirmation of the insertion depth and in vivo position of the middle tube body 71 during surgery. In some embodiments, at least one middle tube radiopaque marker 79 can be positioned on the middle tube pull-ring section 73 and/or the middle tube deflection section 74 of the middle tube body 71 to indicate the relative positions of the middle tube and the outer tube. In some embodiments, the middle tube radiopaque marker 79 can be a tube segment with platinum material added.

The middle tube handle 72 is connected to the proximal end of the middle tube body 71 and is equipped with a middle tube deflection adjustment assembly 721 for regulating the curvature of the middle tube body 71. The middle tube deflection assembly 721 comprises a middle tube knob and a middle tube threaded component. Specifically, in some embodiments, the detailed structure of the middle tube knob is identical to that of the outer tube knob 6211. The middle tube threaded component is sleeved over the middle tube body 71 and is connected to the middle tube deflection member 711. The middle tube knob is configured to rotate and drive the middle tube threaded component to move axially, thereby pulling the middle tube deflection member 711. The middle tube knob is threadedly connected to the middle tube threaded component. By rotating the middle tube knob on the middle tube handle 72, the threaded structure enables the middle tube knob to drive the middle tube threaded component to move axially forward or backward, thus pulling the middle tube deflection member 711 connected to it and controlling the deflection of the middle tube body 71.

Preferably, in some embodiments, a middle tube stress diffusion tube 77 is also provided at the junction between the middle tube handle 72 and the middle tube body 71. The middle tube stress diffusion tube 77 is configured to prevent stress concentration at the connection point between the two components, which could otherwise lead to easy bending. **In** some embodiments, the middle tube handle 72 is equipped with a first middle tube Luer three-way valve 722. In some embodiments, a protective sheath 78 for the valve clip device 5 is also provided on the middle tube body 71, and a second Luer three-way valve 723 is installed on the protective sheath 78. Both the first Luer three-way valve 722 and the second Luer three-way valve 723 are used for connecting to a syringe to facilitate flushing of the middle tube assembly 7 before and after use.

In some embodiments, a middle tube hemostasis valve is also disposed within the middle tube handle 72. The middle tube hemostasis valve is configured to selectively seal the cavity of the middle tube body 71 to minimize blood loss and prevent air from entering the delivery system and forming an air embolism.

It should be noted that, as shown in Figure 49, both the outer tube assembly 6 and the middle tube assembly 7 in this embodiment possess deflection adjustment capabilities. The combined assembly of the outer tube assembly 6 and the middle tube assembly 7 enables layered and segmented control for deflection adjustment, achieving three-dimensional deflection adjustment at the distal end of the delivery system. This further enhances the operational flexibility of the delivery system, facilitating path control in complex and curved cardiovascular passages with simple operation and reducing potential damage during surgery. In this embodiment, the guiding catheter assembly, the middle tube assembly 7, the inner tube assembly 2, and the manipulation components are separately configured and used in coordination, facilitating assembly, disassembly, cleaning, replacement, and reassembly.

The specific process for delivering the valve clip device to the designated position in the heart using the delivery system in this embodiment is as follows:
First, a guidewire is inserted through the blood vessel to the designated position in the heart. Then, the guide catheter 8 is advanced along the guidewire in coordination with the outer tube assembly 6 to reach the heart, establishing a delivery pathway.

After the delivery pathway is established, the guidewire and the guide catheter 8 are withdrawn. Subsequently, the combined assembly of the middle tube assembly 7 and the inner tube assembly 2, along with the valve clip device 5, are delivered to the heart through the cavity of the outer tube assembly 6.

During the delivery process, the curvature of the outer tube body 61 and the middle tube body 71 can be adjusted separately or simultaneously using the outer tube deflection assembly 621 and the middle tube deflection assembly 721, respectively. The delivery path can be adjusted by rotating the middle tube or outer tube handle separately or simultaneously to accurately reach the designated position in the heart.

The control handle 20 is configured to manipulate the manipulation wire 33, thereby controlling the opening and closing of the capturing members 51 of the valve clip device 5.

The control handle 20 can also be configured to control the rotation of the manipulation shaft 31 relative to the main tube layer 213. By controlling the rotation of the manipulation shaft 31, the opening and closing of the closure members 52 of the valve clip device 5 can be controlled.

After the valve clip device 5 has successfully clamped the heart valve, the control handle 20 is configured to detach the connecting portion 311 of the manipulation shaft 31 and the manipulation wire 33 from the valve clip device, leaving the valve clip device as an implant inside the human body.

In this context, directional terms such as front, rear, upper, and lower are defined based on the positions of the components in the drawings and their relative positions to each other, solely for the purpose of clarity and convenience in expressing the technical solution. It should be understood that the use of these directional terms does not limit the scope of protection claimed in this application.

The features described in the above embodiments and their variations can be combined with each other as long as there is no conflict.

The above disclosure represents only a preferred embodiment of the present disclosure and should not be construed as limiting the scope of the disclosure's rights. Therefore, equivalent changes made in accordance with the claims of the present disclosure still fall within the scope of the disclosure.

## Claims

1. A control system for an interventional device, comprising a control handle, wherein the
control handle comprises:
a control assembly (3) comprising a manipulation shaft (31) for controlling a distal instrument and a driving assembly for driving the manipulation shaft (31), wherein the driving assembly comprises a drive shaft (34), which comprises a control shaft connection end (341) at the distal end, the control shaft connection end (341) transmits torque and axial force to the manipulation shaft (31);
a housing assembly (1) comprising an outer housing (11), wherein the outer housing (11) comprises a through-hole at a distal end for an inner tube assembly (2) to extend out of the outer housing (11), and a knob annular groove (115) at the proximal end;
wherein the driving assembly further comprises a knob (35) and a switching mechanism, the knob (35) is rotationally connected to the knob annular groove (115), the drive shaft (34) comprises a threaded connection portion at the proximal end, and the knob (35) is provided with an internal thread that matches the threaded connection portion, the switching mechanism is configured to switch the driving relationship between the knob (35) and the drive shaft (34) to either relative rotation or co-rotation.

2. The control system according to claim 1, wherein the switching mechanism comprises a detent block (37) with a first snap-fit structure (371), the outer surface of the knob (35) is provided with a second snap-fit structure (351), the detent block (37) is sleeved over the drive shaft (34) and is allowed to slides between a first position and a second position relative to the drive shaft (34);
when the detent block (37) is located at the first position, the first snap-fit structure (371) engages with the second snap-fit structure (351) to restrict a rotation of the knob (35) relative to the detent block (37);
when the detent block (37) is located at the second position, the first snap-fit structure (371) disengages from the second snap-fit structure (351), allowing the knob (35) to rotate relative to the detent block (37).

3. The control system according to claim 2, wherein one of the first latching structure (371) and the second latching structure (351) is a key structure, and the other is a keyway structure, the key structure couples with the keyway structure at the first position to restrict the rotation of the knob (35) relative to the detent block (37).

4. The control system according to claim 2, wherein the switching mechanism further comprises a clutch block (36) and a spring (362), a gear block annular groove (114) is provided within the outer housing (11), the gear block annular groove (114) restricts a rotation of the detent block (37) and guides a axial sliding of the detent block (37) relative to the outer housing (11);
a proximal end of the clutch block (36) is provided with a first compression structure (361), and a distal end of the detent block (37) is provided with a second compression structure (372), the spring (362) provides a biasing force to the clutch block (36) such that the first compression structure (361) presses against the second compression structure (372).

5. The control system according to claim 4, wherein the switching mechanism further comprises a toggle plate (38) that is axially fixed to the detent block (37), the toggle plate (38) comprises a first snap (381), and a second snap (116) that matches the first snap (381) is provided within the outer housing (11), when the toggle plate (38) moves the detent block (37) to the second position, the first snap (381) engages with the second snap (116) to prevent the detent block (37) from move back to the first position.

6. The control system according to claim 5, wherein the first compression structure (361) and the second compression structure (372) are configured such that when the detent block (37) rotates, a biasing force is transferred to the first compression structure (361) through the second compression structure (372) to drive the clutch block (36) to move distally.

7. The control system according to claim 6, wherein the second compression structure (372) is a protruding tooth, and the first compression structure (361) is a latching tooth that matches the protruding tooth.

8. The control system according to claim 2, wherein the drive shaft (34) comprises a transmission cavity section (343) having a transmission guide surface (344), the detent block (37) is provided with a gear guide face (374) that matches the transmission guide face (344), when the detent block (37) is located at either the first position or the second position, the gear guide face (374) contacts with the transmission guide face (344) to restrict a movement of the drive shaft (34) relative to the detent block (37).

9. The control system according to claim 1, wherein a manipulation shaft positioning sleeve (32) is fixedly sleeved over a proximal end of the manipulation shaft (31), the control shaft connection end (341) comprises a shaped elongated hole, and an outer surface of the manipulation shaft positioning sleeve (32) matches an inner surface of the shaped elongated hole to restrict relative rotation between the manipulation shaft (31) and the drive shaft (34);
the manipulation shaft positioning sleeve (32) and the shaped elongated hole are configured for relative axial sliding, the shaped elongated hole is longer than the manipulation shaft positioning sleeve (32), and an inner diameter of a manipulation shaft annular groove (134) is smaller than a maximum outer diameter of the manipulation shaft positioning sleeve (32).

10. The control system according to claim 9, wherein a floating elastic member (320) is provided between a proximal end of the manipulation shaft positioning sleeve (32) and a bottom surface of the shaped elongated hole.

11. The control system according to claim 10, wherein the floating elastic member (320) is a spring, when the floating elastic member (320) is in its natural state, a distal end of the manipulation shaft positioning sleeve (32) is located within the shaped elongated hole.

12. The control system according to claim 2, wherein the housing assembly (1) comprises an outer housing (11) and a moving seat (13) located within the outer housing (11), the moving seat (13) comprises an inner cavity (131), and the control shaft connection end (341) is positioned within the inner cavity (131) and rotates relative to the moving seat (13).

13. The control system according to claim 12, wherein the moving seat (13) further comprises an inner guide face (132) located within the inner cavity (131), a first observation window (136) is provided on the moving seat (13);
a second observation window (117) corresponding to the first observation window (136) is provided on the outer housing (11);
an indicating part (4) is provided with threaded features, the indicating part (4) is threadedly engaged with the control shaft connection end (341), and an outer surface of the indicating part (4) is slidably connected to the inner guide face (132) and -relatively fixed in the rotational direction, the indicating part (4) serves an indicating function in conjunction with the first observation window (136) and the second observation window (117).

14. The control system according to claim 13, wherein the inner cavity (131) comprises an inner end surface (1311), the inner end surface (1311) cooperates with both ends of the control shaft connection end (341) to restrict the control shaft connection end (341) within the inner cavity (131).

15. The control system according to claim 13, wherein the moving seat (13) is a split-type, detachable housing.

16. The control system according to claim 13, wherein the moving seat (13) comprises a manipulation shaft annular groove (134) and a drive shaft annular groove (135);
the manipulation shaft annular groove (134) and the drive shaft annular groove (135) are coaxially arranged, an inner diameter of the manipulation shaft annular groove (134) is greater than or equal to an outer diameter of the manipulation shaft (31), an inner diameter of the drive shaft annular groove (135) is greater than an outer diameter of the driving connection section of the drive shaft (34) and smaller than an outer diameter of the control shaft connection end (341).

17. The control system according to claim 12, wherein the moving seat (13) further comprises an outer guide face (133), the outer housing (11) comprises a moving seat guide rail (111), and the outer guide face (133) slidably engages with the moving seat guide rail (111).

18. The control system according to claim 1, wherein it comprises a positioning and guiding mechanism, the positioning and guiding mechanism comprises an inner tube assembly (2) having an inner tube (21) and a sleeve (24) for guiding a manipulation wire (33), the inner tube (21) comprises an inner layer (211) and an outer layer (212), the inner layer (211) is provided with a cavity (2111) for the manipulation shaft (31) to pass through, and the outer layer (212) is provided with a channel (215) for the manipulation wire (33) to pass through, a proximal end of the inner layer (211) extends beyond the outer layer (212), and an positioning sleeve (22) is fixedly sleeved over at least part of the extending section;
the housing assembly (1) comprises an outer housing (11) and a fixed seat (12) disposed within the outer housing (11), the fixed seat (12) comprises a positioning sleeve slot (121) and two sleeve guide slots (122) located on both sides of the positioning sleeve slot (121), the two sleeve guide slots (122) taper from a proximal end to a distal end, the positioning sleeve slot (121) matches the inner tube positioning sleeve (22), and the two sleeve guide slots (122) match the sleeve (24).

19. The control system according to claim 18, wherein a distal end of the fixed seat (12) is further provided with an annular groove (123) for the inner layer (211) to pass through, the annular groove (123) communicates with the positioning sleeve slot (121), and an inner diameter of the annular groove (123) is larger than an inner diameter of the inner layer (211) but smaller than an inner diameter of the inner tube positioning sleeve (22).

20. The control system according to claim 18, wherein the fixed seat (12) is of a split-type, detachable structure.

21. The control system according to claim 18, wherein the outer housing (11) is further provided with a guide rail (112), the inner tube assembly (2) further comprises a push-pull rod (23) slidably disposed on the guide rail (112), and the push-pull rod (23) is fixedly connected to a distal end of the manipulation wire (33).

22. The control system according to claim 21, wherein the inner tube assembly (2) further comprises a support tube (25) disposed within the inner cavity of the push-pull rod (23), and the support tube (25) is fixedly connected to the sleeve (24).

23. A delivery system for an interventional device, wherein it comprises a control system according to any one of claims 1-22, a guiding catheter assembly, a middle tube assembly (7), an inner tube assembly (2), and a manipulation shaft (31);
the guiding catheter assembly comprises an outer tube assembly (6) having an outer tube body (61), an outer tube deflection member (611) is provided within the outer tube body (61) to control the deflection of at least part of the outer tube body (61);
the middle tube assembly (7) comprises a middle tube body (71) having a middle tube deflection member (711), the middle tube deflection member (711) is configured to control the deflection of at least part of the middle tube body (71), the middle tube body (71) accommodates the inner tube (21), and the outer tube body (61) accommodates the middle tube body (71);
the inner tube assembly (2) comprises an inner tube (21), a manipulation wire (33) is provided within the inner tube (21), the manipulation wire (33) is configured to control the capture of a capturing member (51) of a valve clamping device (5);
the manipulation shaft (31) is disposed within the cavity (2111) of the inner tube (21), a distal end of the manipulation shaft (31) is provided with a connecting portion (311) that is connected to the valve clamping device (5), the manipulation shaft (31) rotates relative to the inner tube (21) to control a closure member (52) of the valve clamping device (5) to open and close.

24. The delivery system according to claim 23, wherein the outer tube body (61) is provided, from an inside to an outside along its radial direction, with an inner lining layer (612) and an outer tube body coating layer (613), at least one outer tube support layer (614) is embedded within the outer tube body coating layer (613), at least one outer tube deflection member liner tube (615) is provided within the outer tube body coating layer (613), and the outer tube deflection member (611) is disposed within the outer tube deflection member liner tube (615).

25. The delivery system according to claim 24, wherein the outer tube body (61) comprises, from a distal end to a proximal end, a deflection tip (63), a pull-ring section (64), a deflection section (65), and a main body section (67), the pull-ring section (64) is connected to a distal end of the outer tube deflection member (611), and a proximal end of the outer tube deflection member (611) is connected to an outer tube handle (62), the hardness of the deflection section (65) is less than that of the main body section (67);
preferably, at least one transition section (66) is provided between the deflection section (65) and the main body section (67), with the hardness of the transition section (66) being greater than that of the deflection section (65) but less than that of the main body section (67).

26. The delivery system according to claim 25, wherein the outer tube handle (62) is provided with an outer tube deflection assembly (621), which comprises an outer tube knob (6211) and an outer tube threaded member (6212), the outer tube threaded member (6212) is sleeved over the outer tube body (61) and is connected to the outer tube deflection member (611), the outer tube knob (6211) is configured to rotate and drive the outer tube threaded member (6212) to move axially, thereby pulling the outer tube deflection member (611).

27. The delivery system according to claim 24, wherein the middle tube body (71) is provided, along its radial direction, with a middle tube inner liner layer and a middle tube body covering layer, at least one middle tube support layer is embedded within the middle tube body covering layer, at least one middle tube deflection member liner tube is provided within the middle tube body covering layer, and the middle tube deflection member is disposed within the middle tube deflection member liner tube.

28. The delivery system according to claim 27, wherein the middle tube body (71) comprises, from a distal end to a proximal end, a middle tube pull-ring section (73), a middle tube deflection section (74), a middle tube compliant section (75), and a middle tube main body section (76), the middle tube pull-ring section (73) is connected to a distal end of a middle tube deflection member (711), and a proximal end of the middle tube deflection member (711) is connected to a middle tube handle (72), the hardness of the middle tube deflection section (74) is less than that of the middle tube compliant section (75), and the hardness of the middle tube compliant section (75) is less than that of the middle tube main body section (76);
preferably, at least one middle tube transition section (750) is provided between the middle tube main body section (76) and the middle tube compliant section (75), and between the middle tube compliant section (75) and the middle tube deflection section (74), the hardness of the middle tube transition section (750) is less than that of the middle tube main body section (76).

29. The delivery system according to claim 28, wherein the middle tube handle (72) is provided with a middle tube deflection assembly (721), which comprises a middle tube knob and a middle tube threaded member, the middle tube threaded member is sleeved over the middle tube body (71) and is connected to the middle tube deflection member (711), the middle tube knob is configured to rotate and drive the middle tube threaded member to move axially, thereby pulling the middle tube deflection member (711).

30. The delivery system according to claim 23, wherein the inner tube (21) is connected to a control handle (20) of the control system, and the control handle (20) is provided with a joystick (221) connected to the manipulation wire (33).

31. The delivery system according to claim 23, wherein the inner tube (21) is provided, from an inside to an outside along its radial direction, with an inner layer (211) and an outer layer (212), at least one channel (215) is provided within the outer layer (212), and the manipulation wire (33) is disposed within the at least one channel (215).

32. The delivery system according to claim 23, wherein the guiding catheter assembly further comprises a guide catheter (8), which is detachably disposed within the outer tube body (61), the guide catheter (8) comprises a main body portion (81) and a guiding head (82) that are connected to each other, the outer diameter of the guiding head (82) gradually decreases from an end connected to the main body portion (81) to another endaway from
the main body portion (81).
